# EUROPEAN PATENT SPECIFICATION

(11) **EP 0 302 644 B1**
(45) Date of publication and mention of the grant of the patent: **29.01.1997**
(21) Application number: 88306836.3
(22) Date of filing: 25.07.1988
(51) Int. Cl.: C07D 473/00

(54) **Purine compounds and their preparation**
Purinverbindungen und ihre Herstellung
Dérivés de purine et leur préparation

(30) Priority: 01.08.1987 GB 8718283; 13.06.1988 GB 8813926
(43) Date of publication of application: 08.02.1989
(73) Proprietor: BEECHAM GROUP PLC, Brentford, Middlesex TW8 9EP (GB)
(72) Inventor: Grinter, T. J. SmithKline Beecham Pharmaceuticals, Tonbridge, Kent TN11 9AN (GB); Geen, G. R. SmithKline Beecham Pharmaceuticals, Harlow, Essex CM19 5AD (GB); Parratt, M. J. SmithKline Beecham Pharmaceuticals, Epsom Surrey KT18 5XQ (GB)
(74) Representative: Tocher, Pauline

(56) References cited:
- EP-A- 0 141 927
- EP-A- 0 182 024
- ORGANIC SYNTHESIS, VOL. 60, 1981, PAGES 66-71

## Description

The present invention relates to a chemical process for the preparation of novel compounds which are useful intermediates in the preparation of pharmaceutically active compounds, and to novel intermediates used in that process.

EP-A-0 141 927 and EP-A-0 182 024 describe, inter alia, compounds of formula (A): 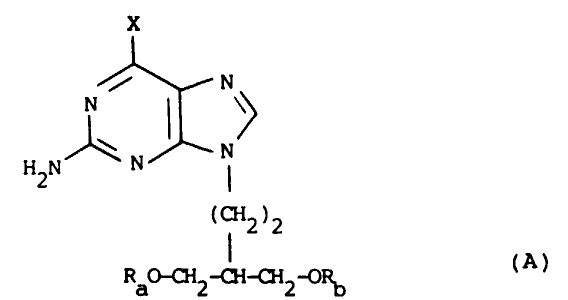 wherein X is hydrogen, hydroxy, chloro, C₁₋₆ alkoxy or phenyl C₁₋₆ alkoxy and Rₐ and R_{b} are hydrogen, including acyl and phosphate derivatives thereof.

The above publications disclose a process for the preparation of compounds of formula (A) which involves the reaction of purine derivatives, including compounds of formula (B): 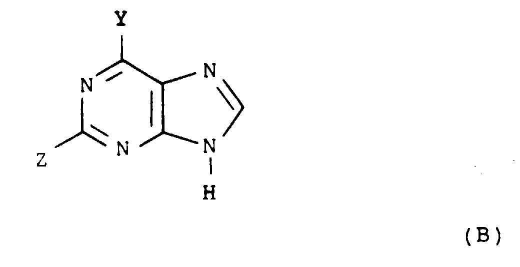 wherein Y is chloro, hydroxy, C₁₋₆ alkoxy, phenyl C₁₋₆ alkoxy or amino, and Z is chloro, amino or acylamino, with compounds of formula (C): 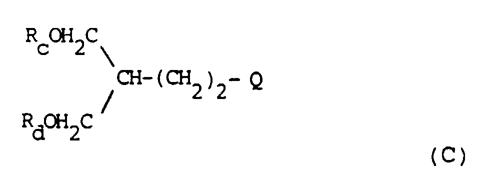 in which R_{c} and R_{d} are each independently acyl or together form a cyclic acetal or cyclic carbonate group and Q is a leaving group such as, chlorine, bromine or iodine, preferably iodine.

This process has the disadvantage that compounds of formula (C) are not readily available and must be prepared individually via multi-stage syntheses.

A new process for the preparation of compounds of formula (A) has now been discovered which uses a readily available or easily prepared starting material in place of the intermediates of formula (C).

According to the present invention there is provided a process for the preparation of a compound (A): 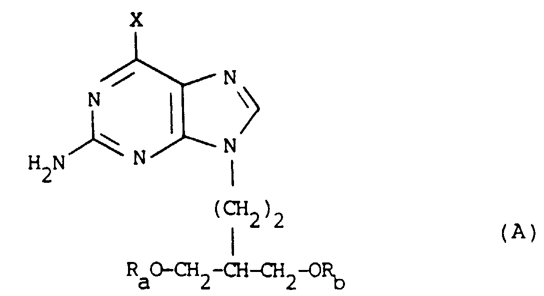 wherein:
X is hydrogen, hydroxy, chloro, C₁₋₆ alkoxy or phenyl C₁₋₆ alkoxy; and Rₐ and R_{b} are hydrogen, or acyl or phosphate derivatives thereof, which process comprises:
(i) the preparation of a compound of formula (I): 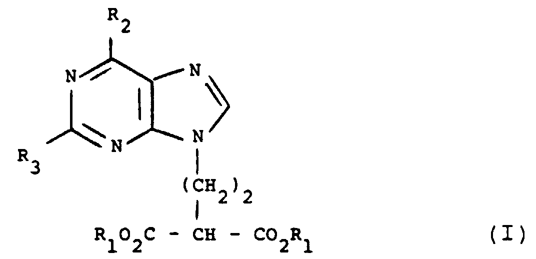 wherein R₁ is C₁₋₆ alkyl, or phenyl C₁₋₆ alkyl in which the phenyl group is optionally substituted; R₂ is hydrogen, hydroxy, chlorine, C₁₋₆ alkoxy, phenyl C₁₋₆ alkoxy or amino; and R₃ is halogen, C₁₋₆ alkylthio, C₁₋₆ alkylsulphonyl, azido, an amino group or a protected amino group, which preparation comprises the reaction of a compound of formula (II): 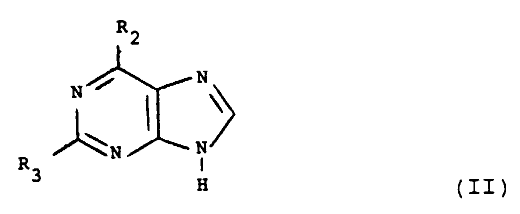 wherein R₂ and R₃ are as defined for formula (I) with:
   (a), a compound of formula (III): 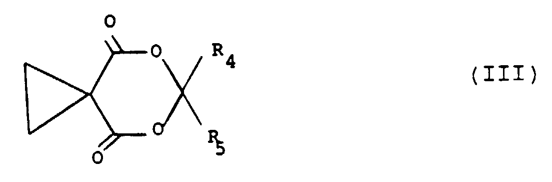 wherein R₄ and R₅ are independently hydrogen, C₁₋₆ alkyl, or phenyl, or R₄ and R₅ together are C₅₋₇ cycloalkyl, to give a compound of formula (IV): 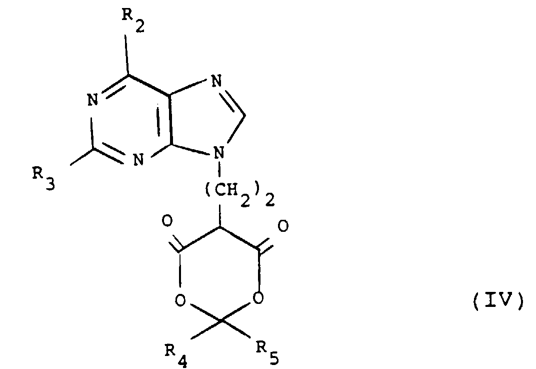 or
   (b), a compound of formula (V): 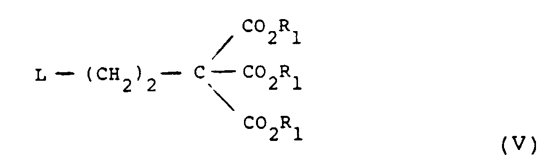 wherein L is a leaving group and R₁ is as defined for formula (I), to give a compound of formula (VI): 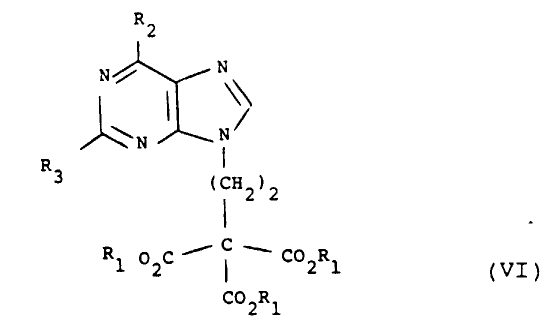 and thereafter converting the intermediate compound of formula (IV) to a compound of formula (I) via transesterification, or the intermediate compound of formula (VI) to a compound of formula (I) via decarboxylation, and, as necessary or desired, interconverting variables R₁, R₂ and R₃ to further values of R₁, R₂ and R₃;
   (ii) the conversion of the resulting compound of formula (I) to a compound of formula (A) by converting variable R₃, when other than amino, to amino, reducing the ester groups CO₂R₁ to CH₂OH and optionally forming acyl or phosphate derivatives thereof, and as necessary or desired converting variable R₂ in the compound of formula (I) to variable X in the compound of formula (A).

As used herein, the term C₁₋₆ alkyl includes groups in which the alkyl moiety is straight or branched, favourably contains 1 to 4 carbon atoms and is preferably methyl. Substituents for phenyl when optionally substituted include one or two of hydroxy, C₁₋₆ alkyl, C₁₋₆ alkoxy and halogen, such as fluoro, chloro, bromo and iodo.

Values for X in compounds of formula (A) include hydrogen, hydroxy and C₁₋₆ alkoxy, for example methoxy. When X is hydroxy it will be appreciated that compounds of formula (A) exist in more than one tautomeric form.

Values for Rₐ and R_{b} in compounds of formula (A) include hydrogen and acyl such as C₂₋₅ alkanoyl, for example acetyl.

Values for R₁ in compounds of formula (I) include C₁₋₄ alkyl, for example methyl and ethyl.

Values for R₂ in compounds of formula (I) include hydrogen, chlorine, and C₁₋₄ alkoxy, for example methoxy.

Suitable values for R₃ when a protected amino group include C₂₋₅ alkanoylamino such as acetylamino or pivaloylamino, aroyl such as benzoyl, and arylmethyl such as benzyl.

Values for R₃ in compounds of formula (I) include amino, halogen for example chlorine, and protected amino such as C₂₋₅ alkanoylamino, for example acetylamino.

When R₂ in compounds of formula (II) is hydrogen, examples of R₃ include halogen for example chlorine, and amino. When R₂ in compounds of formula (II) is chlorine, examples of R₃ include halogen for example chlorine, amino, and acetylamino. Preferably R₂ in compounds of formula (II) is chlorine and R₃ in compounds of formula (II) is amino.

In process variant (a), values for R₄ and R₅ in compounds of formula (III) include hydrogen, C₁₋₄ alkyl for example methyl, phenyl and cyclohexyl. Preferably both R₄ and R₅ are methyl or one of R₄ and R₅ is hydrogen and the other is phenyl, C₁₋₄ alkyl such as methyl, or R₄ and R₅ together are cyclohexyl.

In process variant (b), the leaving group L in compounds of formula (V) is suitably a halogen atom, preferably bromine. Variable R₁ is preferably chosen such that a group CO₂R₁ is readily displaced from the intermediate compound of formula (VI) by decarboxylation. Preferably R₁ is an ethyl group.

The reaction of a compound of formula (II) with a compound of formula (III) according to process variant (a) or a compound of formula (V) according to process variant (b) may be carried out in an inert solvent for example dimethylformamide, dimethylsulphoxide or acetonitrile, preferably dimethylformamide, in the presence of an inorganic or organic base, over a temperature range from 0°C to the boiling point of the solvent. Examples of inorganic bases include alkali metal hydrides, alkali metal carbonates such as sodium or potassium carbonate and preferably potassium carbonate. Suitable organic bases are 1,8-diazabicyclo[5.4.0]undec-7-ene and tetramethyl guanidine. The reaction conditions selected for the preparation of intermediate compounds of formulae (IV) and (VI) according to process variant (a) and (b) respectively may result in the isolation of these intermediate compounds as salts. For example, use of an alkali metal carbonate in process variant (a) may result in the isolation of the intermediate compound of formulae (IV) as the corresponding alkali metal salt.

Compounds of formula (I) may be obtained by transesterification of the compound of formula (IV) under conventional conditions, for example via acid catalysed reaction with the appropriate alcohol of formula (VII):

R₁-OH (VII)

wherein R₁ is as defined in formula (I). Advantageously C₁₋₆ aliphatic alcohols, for example methyl alcohol or ethyl alcohol, are used. An inert solvent may be added, if required.

The reaction may be carried out at temperatures ranging from ambient to the boiling point of the alcohol or inert solvent, if present.

Alternatively, compounds of formula (I) wherein R₂ is OR₁ may be obtained directly from compounds of formula (IV) wherein R₂ is chlorine via treatment with an alcohol of formula (VII), transesterification and displacement of chlorine by the group OR₁ taking place in the same reaction.

Compounds of formula (I) may be obtained by monodecarboxylation of the intermediate compounds of formula (VI). Decarboxylation may be carried out under conventional conditions, for example, by stirring at ambient temperature in the presence of a base such as sodium ethoxide in a solvent such as ethanol or tetrahydrofuran.

Compounds of formula (I) in which R₂ is chlorine, made by process variant (b), may be treated with an alcohol of formula (VII), as described above, to give a compound of formula (I) in which R₂ is OR₁. It will be appreciated that R₁ in compounds of formula (I) made by process variant (b) may be transesterified to further values of R₁ via reaction with an alcohol of formula (VII).

Intermediate compounds of formula (IV) and formula (VI) in which R₂ is chlorine may be hydrogenolysed to give intermediate compounds of formula (IV) and formula (VI) respectively in which R₂ is hydrogen, preferably by catalytic reduction using a noble metal catalyst, for example palladium on charcoal, in the presence of hydrogen or a hydrogen source such as ammonium formate, in an alcoholic solvent, preferably methanol or ethanol.

Variable R₃ in compounds of formula (I) may be converted to further values of R₃ using procedures conventionally practised in purine chemistry. For example, an amine protecting group such as arylmethyl may be removed by hydrogenolysis. Where the intermediate compounds of formulae (IV) and (VI) are subjected to hydrogenolysis reactions as described above, the protecting group will be removed at this intermediary stage. Similarly, variable R₃ may be converted from azido to amino by catalytic reduction, and an R₃ halogen, alkylthio or alkylsulphonyl group may be converted to an R₃ amino group by aminolysis using, for example, ammonia.

Variables R₁ and R₂ may of course be susceptible to the reaction conditions chosen for interconversion of variable R₃. It will be apparent to the skilled chemist that the process variant [(a) or (b)] followed, and the stage in the reaction sequence at which the transformation of variables, where necessary or desired, is carried out, may be chosen to suit the variables R₁, R₂ and R₃ required in the compound of formula (I).

The compounds of formulae (I), (IV) and (VI) are novel compounds and form part of the present invention. Compounds of formulae (I), (IV) and (VI) may form salts and solvates such as hydrates, and the invention also extends to these forms. Some of the compounds of formula (III) are known compounds. The compound of formula (III) in which R₃ and R₄ are methyl may be prepared according to the procedure described in Organic Syntheses, (Vol. 60, P. 66). Other compounds of formula (III) may be prepared by analogous procedures. The alcohols of formula (VII) are known compounds or are prepared by analogous procedures to those used to prepare known compounds of formula (VII).

Certain compounds of formula (V) are known compounds. The compound of formula (V) in which L is bromine and R₁ is ethyl may be prepared from commercially available triethyl methanetricarboxylate according to the procedure described by H. Rapoport et al, [Journal of Organic Chemistry, 44, 3492 (1979)]. Other compounds of formula (V) may be prepared by an analogous procedure. Methane tricarboxylate derivatives may be prepared by standard methods from the corresponding malonic acid derivatives.

Purine derivatives of formula (II) are generally known compounds and their preparation is described in the prior art relating to purine chemistry. The compound of formula (II) in which R₂ is chlorine and R₃ is an amino group is 2-amino-6-chloropurine, utilised in the process of the Examples disclosed in EP-A-0 141 927.

The compounds of formula (I) in which R₂ is hydrogen, hydroxy, chloro, C₁₋₆ alkoxy or phenyl C₁₋₆ alkoxy and R₃ is an amino group may be reduced under conventional conditions, for example using sodium borohydride, to the compounds of formula (A) in which X is hydrogen, hydroxy, chloro, C₁₋₆ alkoxy or phenyl C₁₋₆ alkoxy and Rₐ and R_{b} are hydrogen. The compound of formula (A) in which X is hydroxy and Rₐ and R_{b} are hydrogen may be obtained under conventional hydrolysis conditions, for example in aqueous sodium hydroxide solution, from compounds of formula (A) in which X is C₁₋₆ alkoxy or phenyl C₁₋₆ alkoxy and Rₐ and R_{b} are hydrogen. Compounds of formula (A) in which X is hydrogen or hydroxy and Rₐ and R_{b} are hydrogen may be converted to further compounds of formula (A) in accordance with the procedures described in EP-A-0 182 024 and EP-A-0 141 927.

The following Descriptions and Examples illustrate the process and novel compounds of the invention. The use of the novel compounds of the invention in the preparation of compounds of formula (A) is included by way of illustration.

### Description 1

### 2-Amino-6-chloro-9-[1-(2,2-dimethyl-1,3-dioxane-4,6-dione-5-yl)eth-2-yl]purine potassium salt

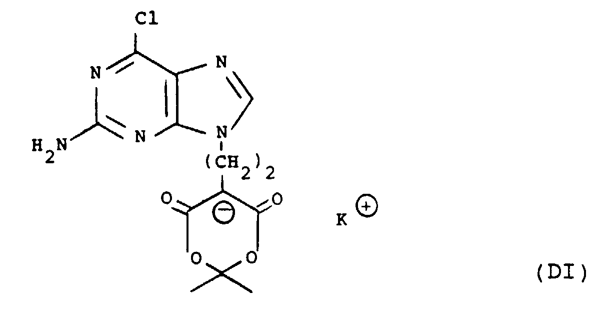

A mixture of 2-amino-6-chloropurine (0.5g, 2.94mmol), 6,6-dimethyl-5,7-dioxaspiro[2.5]octane-4,8-dione (0.5g, 2.94mmol) and potassium carbonate (0.49g, 3.53mmol) in N,N-dimethylformamide (10ml) was stirred at room temperature under dry nitrogen overnight. T.l.c. [chloroform/methanol (2:1)] showed two products, rf= 0.21, 0.34. The mixture was filtered and the filtrate evaporated to leave an oil which was triturated with dichloromethane (5ml) to give a cream coloured solid. The solid was dissolved in chloroform/methanol (3:1) (7.8ml) and column chromatographed on silica (125g) (eluant = chloroform/methanol (3:1) gradually increasing to (1:1)) to give the title compound (0.51g, 46%), rf [chloroform/methanol (2:1)] = 0.34 and 2-amino-6-chloro-7-[1-(2,2-dimethyl-1,3-dioxane-4,6-dione-5-yl)eth-2-yl]purine potassium salt (0.23g, 21%), rf [chloroform/methanol (2:1)] = 0.21

¹H n.m.r. (CD₃OD) of title compound: δ1.50(s,6H,-CH₃), 2.72(t,2H,³J_{HH}=8Hz,-CH₂-), 4.20(t,2H,³J_{HH}=8Hz, >NCH₂-), 8.05(s,1H,H-8).

Decomposition occurs at 290°C.

### Description 2

### 2-Amino-9-[1-(2,2-dimethyl-1,3-dioxane-4,6-dione-5-yl)-eth-2-yl]purine potassium salt

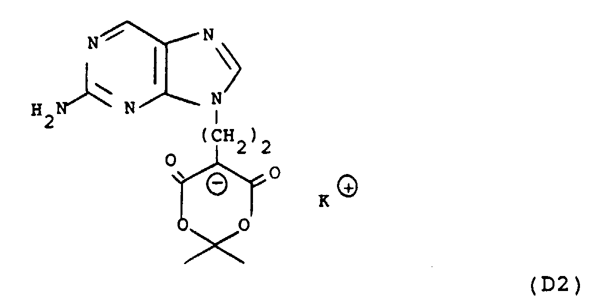

A mixture of 2-amino-6-chloro-9-[1-(2,2-dimethyl-1,3-dioxane-4,6-dione-5-yl)eth-2-yl]purine potassium salt (200mg, 0.53mmol), ammonium formate (148mg, 2.34mmol) and 10% palladium on charcoal (80mg) in methanol (15ml) was heated under reflux under dry nitrogen for 3h. T.l.c. [chloroform/methanol (2:1)] showed one spot, rf=0.15. The mixture was filtered and the filtrate evaporated to leave a gum. The material was not purified further.

¹H n.m.r. (D₂O): δ1.45(s,6H,-CH₃), 2.65(t,2H,³J_{HH}= 8Hz,-CH₂-), 4.20(t,2H,³J_{HH}=8Hz, >NCH₂-), 8 .05(s,1H,H-8), 8.50(s,1H,H-6).

### Description 3

### 6-Phenyl-5,7-dioxaspiro[2.5]octane-4,6-dione

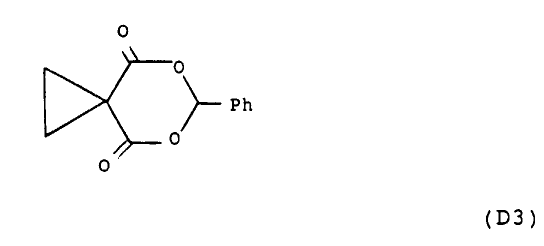

To a suspension of cyclopropane-1,1-dicarboxylic acid (1.0g, 7.69mmol) in a mixture of acetic anhydride (0.86g, 8.45mmol) and benzaldehyde (0.81g, 7.63 mmol) stirred at room temperature was added one drop of concentrated sulphuric acid. The suspension immediately became a light brown solution which, after two minutes turned into a pink solid. The reaction mixture was partitioned between water (100ml) and dichloromethane (2x100ml). The combined organic extracts were dried (MgSO₄), filtered and evaporated to leave a white solid which was triturated with ether/hexane (1:1) (15ml) to leave 0.70g (42%) of white solid material, rf[ether/hexane (3:2)]=0.40, mp 131-131.5°C.

¹H n.m.r (CDCl₃): δ1.90(s,4H,-CH₂-),
6.80(s,1H,-O-CHPhO-), 7.45(brs,5H,-C₆H₅).

### Description 4

### 2-Amino-6-chloro-9-[1-(2-phenyl-1,3-dioxane 4,6-dione-5-yl)eth-2-yl-]purine potassium salt

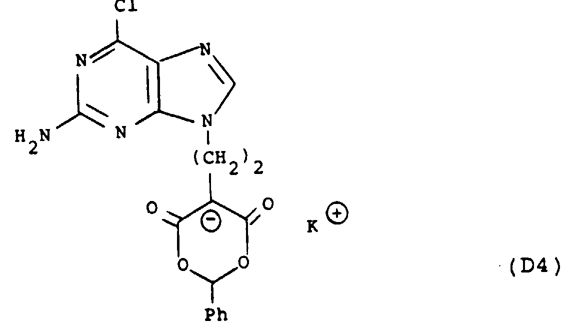

A mixture of 2-amino-6-chloropurine (0.23g, 1.36mmol), 6-phenyl-5,7-dioxaspiro[2.5]octane-4,8-dione (0.30g, 1.37mmol) and potassium carbonate (0.22g, 1.59mmol) in N,N-dimethylformamide (5ml) was stirred at room temperature under dry nitrogen overnight. T.l.c. [chloroform/methanol (2:1)] showed two products, rf= 0.45, 0.55. The mixture was filtered and the filtrate evaporated to leave an oil which was triturated with dichloromethane (15ml) to give a cream coloured solid. The solid was purified by column chromatography on silica (65g) [eluent=dichloromethane/methanol (4:1) gradually increasing to (1:1)] to give the title compound (0.21g, 36%), rf[chloroform/methanol (2:1)]= 0.55 and 2-amino-6-chloro-7-[1-(2-phenyl-1,3-dioxane-4,6-dione-5-yl)eth-2-yl]purine potassium salt (0.14g, 24%), rf[chloroform/methanol (2:1)]=0.45.

¹H n.m.r (D⁶-DMSO): of the title compound: δ2.55 (m,2H,-CH₂-), 4.20(m,2H, >NCH₂-), 6.80(brs,2H,-NH₂), 7.50(brs, 6H, -C₆H₅+-OCHPhO-), 8.10(s,1H,H-8).

### Description 5

### 5,12-Dioxadispiro[2.2.5.2]tridecane-4,13-dione

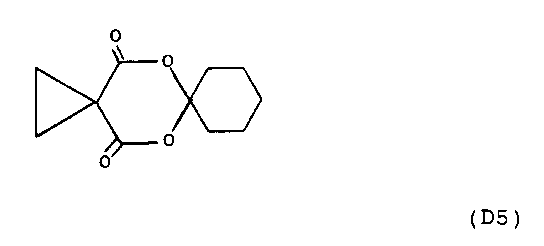

A mixture of cyclopropane-1,1-dicarboxylic acid (13.0g, 0.10mmol), cyclohexanone (10.8g, 0.11mol), acetic anhydride (11.2g, 0.11mol) and concentrated sulphuric acid (5 drops) was stirred at room temperature 0.5h. The mixture soon became a wine-red solution which later darkened to purple. The reaction mixture was partitioned between water (100ml) and ether (2x100ml). The combined organic extracts were washed with brine (50ml), dried (MgSO₄), filtered and evaporated to leave a purple oil plus some solid material. This mixture was purified by column chromatography on silica [eluent=ether/hexane (4:1)] to give the title compound as colourless crystals (2.70g, 13%), m.p. 134.5-135°C.

¹H n.m.r (CDCl₃): δ1.35-2.18(m,10H,cyclohexyl-CH₂-), 1.93(s,4H,cyclopropyl-CH₂-).

### Description 6

### 2-Amino-6-chloro-9-[1-[1,5-dioxaspiro[5.5]undecane-2,4-dione-3-yl]eth-2-yl]purine potassium salt

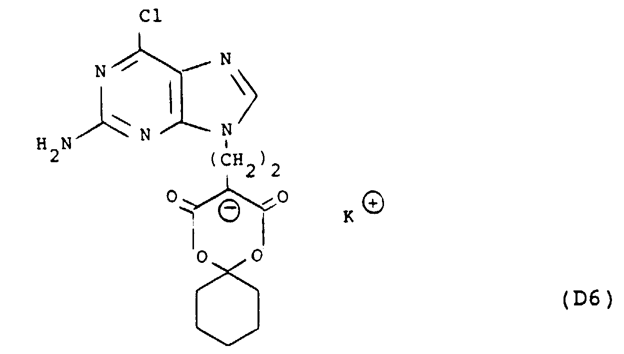

A mixture of 2-amino-6-chloropurine (0.80g, 4.72mmol), 5,12-dioxaspiro[2.2.5.2]tridecane-4,13-dione (1.0g, 4.80mmol) and potassium carbonate (1.0g, 7.23mmol) in N,N-dimethylformamide (10ml) was stirred at room temperature under dry nitrogen for 36h. The mixture was filtered and the filtrate evaporated to give a thick foam (2.7g) which was shown by ¹H n.m.r. analysis to be a mixture of the title compound and 2-amino-6-chloro-7-[1-[1,s-dioxaspiro[5.5]undecane-2,4-dione-3-yl]eth-2-yl]purine potassium salt in the ratio of 2:1 respectively.

¹H n.m.r (D⁶-DMSO) of the title compound:
δ1.25-2.25(m,10H,cyclohexyl-CH₂-), 3.02(m,2H,-CH₂-), 3.78(t,2H,³J_{HH}= 8Hz, >NCH₂-),6.77(brs,2H,-NH₂), 8.13 (s,1H,H-8).

### Description 7

### 2-Acetylamino-6-chloro-9-[1-(2,2-dimethyl-1,3-dioxane-4,6-dione-5-yl]eth-2-yl]purine potassium salt

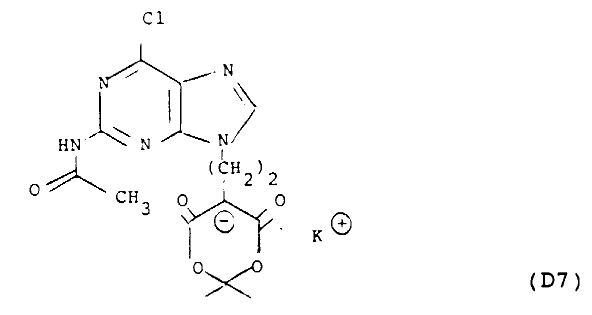

A mixture of 2-acetylamino-6-chloropurinel (1.0g, 4.72mmol), 6,6-dimethyl-5,7-dioxaspiro[2.5]octane-4,8-dione (0.80g, 4.70mmol) and potassium carbonate (0.78g, 5.64mmol) in N,N-dimethylformamide (25ml) was stirred at room temperature under dry nitrogen overnight. T.l.c.[chloroform/methanol(2:1)] showed two products, rf=0.50, 0.65. The mixture was filtered and the filtrate evaporated to leave a light brown gum which was triturated with dichloromethane (30ml) to leave a pale yellow solid (2.0g) which was shown by ¹H n.m.r. analysis to be a mixture of the title compound and 2-acetylamino-6-chloro-7-[1-(2,2-dimethyl-1,3-dioxane-4,6-dione-5-yl]eth-2-yl]purine potassium salt in the ratio of 3:2 respectively.

¹H n.m.r (D⁶-DMSO) of the title compound:
δ1.40(s,6H,-CH₃), 2.25(s,3H,-COCH₃), 2.65(t,2H,³J_{HH}= 8Hz, -CH₂-), 4.20(t,2H,³J_{HH}=8Hz, >NCH₂-), 8.35(s,1H,H-8).

1. W.A. Bowles, F.H. Schneider, L.R. Lewis and R.K. Robins, J. Med. Chem., 6, 471, (1963).

### Description 8

### 2-Amino-9-[1-(2,2-dimethyl-1,3-dioxane-4,6-dione-5-yl)eth-2-yl]purine potassium salt

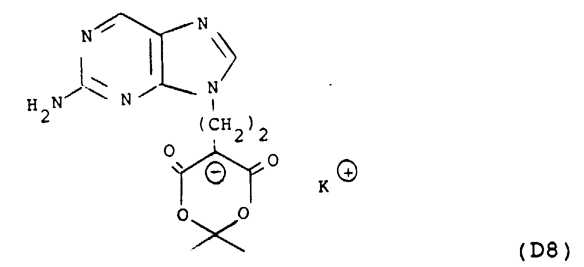

A mixture of 2-aminopurine² (200 mg, 1.48mmol), 6,6-dimethyl-5,7-dioxaspiro[2.5]octane-4,8-dione (252mg, 1.48mmol) and potassium carbonate (245mg, 1.77mmol) in N,N-dimethylformamide (5ml) was stirred at room temperature under dry nitrogen overnight. T.l.c. [chloroform/methanol (2:1)] showed two products, rf=0.23, 0.33. The mixture was evaporated and the residue purified by column chromatography on silica (25g) [eluent=chloroform/methanol (2:1)] to give the the title compound (250mg, 49%), rf[chloroform/methanol (2:1)] = 0.33 and 2-amino-7-[1-(2,2-dimethyl-1,3-dioxane-4,6-dione-5-yl)eth-2-yl]purine potassium salt (150mg, 29%), rf[chloroform/methanol (2:1)]=0.23.

¹H n.m.r (D⁶-DMSO) of the title compound:
δ1.35(s,6H,-CH₃), 2.52(t,2H,³J_{HH}= 8Hz,-CH₂-), 4.20(t,2H,³J_{HH}= 8Hz,>NCH₂-), 8.05(s,1H,H-8), 8.60 (s,1H, H-6).

2. A. Albert and D.J. Brown, J. Chem.Soc., 2060, (1954).

### Description 9

### 2-Chloro-9-[1-(2,2-dimethyl-1,3-dioxane-4,6-dione-5-yl)eth-2-yl]purine potassium salt

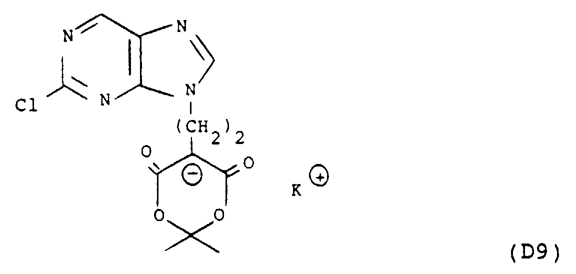

A mixture of 2-chloropurine³ (229mg, 1.48mmol), 6,6-dimethyl-5,7-dioxaspiro[2.5]octane-4,8-dione (252mg, 1.48mmol) and potassium carbonate (245mg, 1.77mmol) in N,N-dimethylformamide (5ml) was stirred at room temperature under dry nitrogen for 66h. T.l.c. [chloroform/methanol (2:1)] showed one spot, rf = 0.45. The mixture was evaporated and the residue purified by column chromatography on silica (35g) [eluent = dichloromethane/methanol (9:1) gradually increasing to (1:1)] to give a white solid (340mg) which was shown by ¹H n.m.r. analysis to be a mixture of the title compound and 2-chloro-7-[1-(2,2-dimethyl-1,3-dioxane-4,6-dione-5-yl)eth-2-yl]purine potassium salt in the ratio of 3:2 respectively.

¹H n.m.r. (D⁶-DMSO) of the title compound:
δ1.55 (s,6H,-CH₃), 2.60 (m, 2H, -CH₂-), 4.45 (m, 2H, >NCH₂-), 8.60 (s, 1H, H-8), 9.10 (s,1H, H-6).

3. J.A. Montgomery, J. Am. Chem. Soc., 78, 1928, (1956).

### Description 10

### 2,6-Dichloro-9-[1-(2,2-dimethyl-1,3-dioxane-4,6-dione-5-yl)eth-2-yl]purine potassium salt

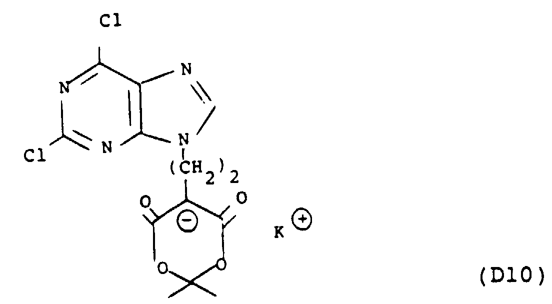

A mixture of 2,6-dichloropurine⁴ (95mg, 0.50mmol), 6,6-dimethyl-5,7-dioxaspiro[2.5]octane-4,8-dione (85mg, 0.50mmol) and potassium carbonate (83mg, 0.60mmol) in N,N-dimethylformamide (2ml) was stirred at room temperature under dry nitrogen overnight. T.l.c. [chloroform/methanol(2:1)] showed one spot, rf = 0.55. The mixture was evaporated and the residue partitioned between water (10ml) and dichloromethane (5ml). The aqueous layer was evaporated to leave a light brown gum which was purified by column chromatography on silica (25g)[eluent = chloroform/methanol (4:1) gradually increasing to (2:1)] to give a colourless glassy material (150mg) which was shown by ¹H n.m.r. analysis to be a mixture of the title compound and 2,6-dichloro-7-[1-(2,2-dimethyl-1,3-dioxane-4,6-dione-5-yl)eth-2-yl]purine potassium salt in the ratio of 3:2 respectively.

¹H n,m.r. (D₂O) of title compound:
δ1.45(s,6H,-CH₃), 2.60(m, 2H, -CH₂-), 4.30 (m, 2H, >NCH₂-), 8.45 (s, 1H, H-8).

4. U.S. Patent No. 3,314,938, 1967.

### Description 11

### 2-Amino-6-chloro-9-(ethyl 2,2-dicarboethoxybutanoate-4-yl)purine

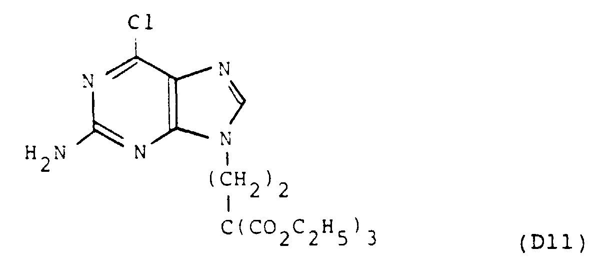

Ethyl 4-bromo-2,2-dicarboethoxybutanoate (93g) was added to a stirred mixture of 2-amino-6-chloropurine (47g) and anhydrous potassium carbonate (57g) in N,N-dimethylformamide (1 dm³) and the resulting mixture stirred at 40°C overnight. The reaction mixture was filtered and the filtrate evaporated. Ethyl acetate (1000 cm³) was added to the residue and the solution washed with water (3 x 500cm³) and brine (500cm³). After drying over magnesium sulphate the solution was evaporated to give a yellow solid. T.l.c. (5% methanol-dichloromethane) showed two products, rf = 0.42, 0.58; corresponding to the N7- and N9- alkylated purines.

Recrystallisation from butan-1-ol (350cm³) gave 43g (37%) of the title compound. Column chromatography on silica (eluant 5% methanol-chloroform) of the filtrate gave a further 22g (19%) of the desired material, m.p. 107-108°C.

¹H n.m.r. (D⁶-DMSO): δ1.20(t,9H,-CH₂CH₃), 2.65(t,2H, -CH₂C〈̶), 4.20(q,6H, -CH₂CH₃), 4.35(t,2H, >N-CH₂), 6.95 (brs, 2H, -NH₂), 8.10 (s,1H, H-8).

### Description 12

### 2-Amino-9-(ethyl 2,2-dicarboethoxybutanoate-4-yl)purine

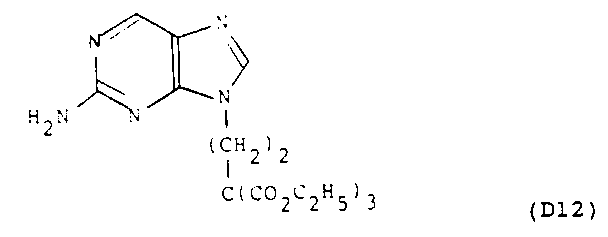

A mixture of 2-amino-6-chloro-9-(ethyl 2,2-dicarboethoxybutanoate-4-yl)purine (21.4g), prepared as in Description 11, ammonium formate (20g) and 5% palladium on charcoal (4g) in methanol (200cm³) was heated under reflux under nitrogen for 2 hours. After cooling, the mixture was filtered and the filtrate evaporated to a gum. The gum was dissolved in water (400cm³), extracted with chloroform (3 x 200cm³) and the combined extract dried over magnesium sulphate. Filtration and evaporation gave the title compound as an oil 18.7g (95%) which slowly crystallised on standing. m.p. 58-60°C.

¹H n.m.r. (D⁶-DMSO): δ1.20(t,9H, -CH₂CH₃), 2.65 (t, 2H, -CH₂C〈̶), 4.15(q,6H, -CH₂CH₃), 4.35(t,2H, >N-CH₂), 6.50(brs, 2H, -NH₂), 7.95(s,1H,H-8), 8.65(s,1H,H-6).

### Example 1

### 2-Amino-9-(methyl 2-carbomethoxybutanoate-4-yl)purine

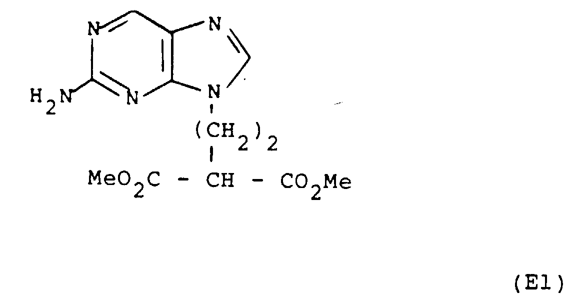

The crude 2-amino-9-[1-(2,2-dimethyl-1,3-dioxane-4,6-dione-5-yl)eth-2-yl]purine potassium salt from Description 2 was suspended in methanol (10ml) saturated with hydrogen chloride, the mixture diluted with methanol (10ml) and stirred overnight at room temperature, giving a clear, colourless solution. T.l.c. [chloroform/methanol (9:1)] showed one spot, rf=0.45. The mixture was evaporated, the residue dissolved in water (20ml) and the solution neutralized using saturated aqueous sodium bicarbonate solution. The aqueous solution was then extracted with dichloromethane (6 x 25ml), the combined extracts dried over magnesium sulphate, filtered and evaporated to leave the title compound (142mg, 82%) as an oil.

¹H n.m.r. (CDCl₃): δ2.40(q,2H,³J_{HH}=8Hz,-CH₂-), 3.35 (t,1H,³J_{HH}=8Hz,>CH-), 3.65(s,6H,-CH₃), 4.15(t,2H, ³J_{HH}=8Hz,>NCH₂-), 5.35(brs,2H,-NH₂), 7.70(s, 1H,H-8), 8.62(s,1H,H-6).

### Example 2

### 2-Amino-6-methoxy-9-(methyl 2-carbomethoxybutanoate-4-yl)purine

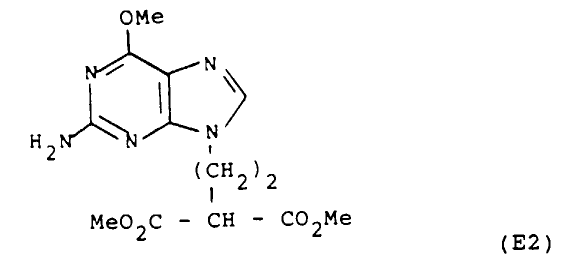

2-amino-6-chloro-9-[1-(2,2-dimethyl-1,3-dioxane-4,6-dione-5-yl)eth-2-yl]purine, potassium salt (1.70g, 4.50mmol), prepared as in Description 1, was suspended in methanol (35ml) saturated with hydrogen chloride, the mixture diluted with methanol (35ml) and stirred at room temperature overnight giving a clear, pale yellow solution. T.l.c. [chloroform/methanol (9:1)] showed one spot, rf=0.65. The mixture was evaporated under reduced pressure, the residue dissolved in water (70ml) and the solution neutralized using saturated aqueous sodium bicarbonate solution. The aqueous solution was then extracted with dichloromethane (5 x 100ml), the combined extracts dried over magnesium sulphate, filtered and evaporated to leave the title compound (1.27g, 87%) as a colourless viscous oil, a sample of which was recrystallised from water to give colourless needles.

m.p. 108-109°C.

¹H n.m.r. (CDCl₃): δ2.47(q,2H,³J_{HH}=7Hz,-CH-); 3.40 (t,1H,³J_{HH}=7Hz,>CH-); 3.75(s, 6H,-CO₂CH₃); 4.10 (s,3H, -OCH₃); 4.20(t,2H,³J_{HH}=7Hz,>NCH₂-); 4.95(brs,2H,-NH₂); 7.60 (s,1H,H-8).

### Example 3

### 2-Amino-9-(ethyl 2-carboethoxybutanoate-4-yl)purine

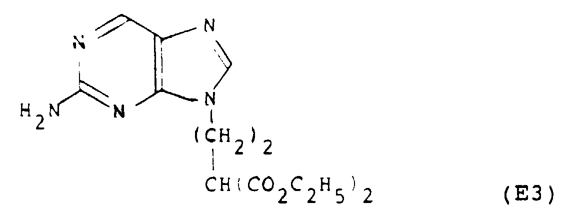

2-Amino-9-(ethyl 2,2-dicarboethoxybutanoate-4-yl)purine (3.93g) in ethanol (25cm³), prepared as in Description 12, was added to a solution of sodium (0.7g) in ethanol (15cm³) and the mixture stirred at ambient temperature for 1 hour. T.l.c. (10% methanol-chloroform) showed one spot, rf = 0.36. The reaction mixture was acidified to pH 3 with dilute hydrochloric acid (ca. 15cm³) and the solvent evaporated. The residue was extracted with dichloromethane (2 x 250cm³) and the combined extract dried over magnesium sulphate. Filtration and evaporation gave a yellow oil.

The oil was dissolved in dichloromethane (ca. 10cm³) and column chromatographed on silica (100g) (eluant 5% methanol-dichloromethane) to give the title compound 1.9g (59%) as an oil which crystallised on standing at ambient temperature. m.p. 65-66°C.

¹H n.m.r. (D⁶-DMSO): δ1.15(t,6H, -CH₂CH₃), 2.35 (q, 2H, -CH₂CH<), 3.50 (t,1H, -CH<), 4.05 (dq, 4H, -CH₂CH₃), 4.20 (m,2H, >N-CH₂), 6.55(brs, 2H, -NH₂), 8.05 (s, 1H, H-8), 8.60 (s,1H, H-6).

### Preparation of Compounds of Formula (A)

### a) 2-Amino-9-(4-hydroxy-3-hydroxymethylbut-1-yl)purine

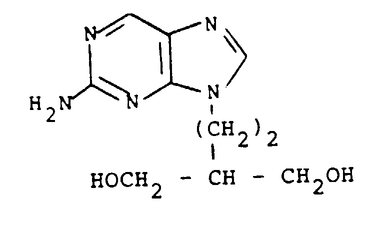

The compound of Example 1, 2-amino-9-(methyl-2-carbomethoxybutanoate-4-yl)purine (0.11g, 0.38mmol) was dissolved in t-butanol (4.5ml) at 60°C under dry nitrogen. Sodium borohydride (84mg, 2.22mmol) was added, the mixture heated to reflux and methanol (0.4ml) added slowly over 2h. The mixture was cooled, water (10ml) was added and the solution was neutralized using dilute aqueous hydrochloric acid. The solution was evaporated to leave a white solid which was column chromatographed on silica (20g) [eluant = chloroform/methanol (2:1)] to give the title compound (50mg, 55%) as a white solid, rf [chloroform/methanol (2:1)]=0.40.

¹H n.m.r.(D₂O): δ1.85(m,3H,-CH₂- + >CH-), 3.65(d,4H,³J_{HH}=5Hz,-CH₂O-), 4.17(t,2H,³J_{HH}=6Hz, >NCH₂-), 8.12(s,1H,H-8), 8.53(s,1H,H-6).

### b) 2-Amino-9-(4-hydroxy-3-hydroxymethylbut-1-yl)purine

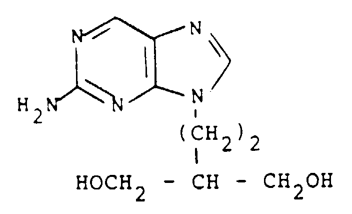

The compound of Example 3, 2-amino-9-(ethyl 2-carboethoxybutanoate-4-yl)purine (3.21g) was dissolved in t-butanol (100cm³) at 60°C. Sodium borohydride (2.3g) was added and the mixture heated to reflux. Methanol (10cm³) was added dropwise over 1 hour with vigorous stirring. The mixture was cooled, water (150cm³) added and the solution neutralised using dilute hydrochloric acid. After evaporation the residue was extracted with methanol (100cm³) and the mixture filtered. The filtrate was evaporated to leave a yellow gum, t.l.c. (35% methanol-chloroform) showed this to be the desired material, rf 0.40. Purification via column chromatography on silica (100g) [eluant = 30% methanol-chloroform] gave the title compound 1.2g (50.5%) as an off-white solid. m.p. 154°C.

¹H n.m.r. (D⁶-DMSO): δ1.45(m, 1H, -CH<), 1.80 (q, 2H, -CH₂CH<), 3.35 (m,2H,-OCH₂), 3.40 (m,2H,-OCH₂-), 4.10 (t,2H, >N-CH₂), 4.40 (t,2H,-OH), 6.50 (brs, 2H,-NH₂), 8.10 (s,1H,H-8), 8.60 (s,1H,H-6).

### c) 9-(4-Acetoxy-3-acetoxymethylbut-1-yl)-2-aminopurine

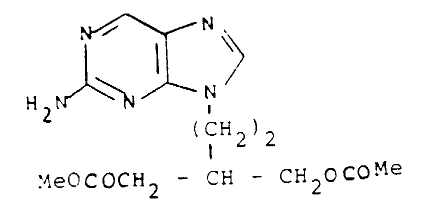

To a suspension of 2-amino-9-(4-hydroxy-3-hydroxymethylbut-1-yl)purine (0.13g, 0.55mmol) in dry tetrahydrofuran (40ml), stirred at room temperature under dry nitrogen were added pyridine (117µl, 1.45mmol) and 4-dimethylaminopyridine (5mg, 41µmol) followed by acetic anhydride (108µl, 1.14mmol). The mixture was stirred at room temperature for 5h. giving a clear, colourless solution. T.l.c. [chloroform/methanol (9:1)] showed the major product to be the title compound, rf = 0.40. Methanol (5ml) was added, the mixture stirred for 5mins. then evaporated to dryness. The residue was partitioned between water (5ml) and chloroform (10ml). The aqueous portion was extracted with chloroform (4x10ml) then the combined organic portions dried over magnesium sulphate, filtered and evaporated to leave a pale yellow glassy material which was column chromatographed on silica (20g) [eluant = chloroform/methanol (19:1)] to give the title compound (0.16g, 91%) as a colourless viscous oil, which was crystallised from n-butanol (0.6ml) to give colourless crystals (118mg., 67%), m.p. 102°C.

¹H n.m.r(CDCl₃): δ1.90(m,3H,-CH₂- + >CH-), 2.00(s,6H,-CH₃), 4.05(d,4H,³J_{HH}=5Hz,-CH₂-O), 4.10(t,2H,³J_{HH}=6Hz,>NCH₂-), 5.35(br.s,2H,-NH₂), 7.70(s,1H,H-8), 8.60(s,1H,H-6).

### d) 2-Amino-6-methoxy-9-(4-hydroxy-3-hydroxymethylbut-1-yl)purine

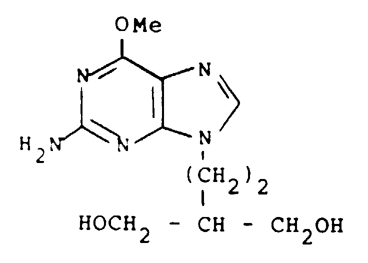

2-Amino-6-methoxy-9-(methyl 2-carbomethoxybutanoate-4-yl)purine (130mg, 0.40mmol) was dissolved in t-butanol (5.8ml) at 60°C under dry nitrogen. Sodium borohydride (100mg, 2.64mmol) was added, the mixture heated to reflux and methanol (0.58ml) added slowly over 2.5h. The mixture was cooled, water (10ml) added and the resulting solution neutralized using dilute aqueous hydrochloric acid. The solution was evaporated under reduced pressure to leave a white solid which was column chromatographed on silica (50g) [eluant - chloroform/methanol (4:1)] to give the title compound (81mg, 76%) as a colourless viscous oil, rf[chloroform/methanol (2:1)]=0.5, a sample of which was crystallised from n-butanol to give a cream-coloured solid, m.p. 84-86°C.

¹H n.m.r.(D₂O): δ1.76(sept.,1H,³J_{HH}-7Hz,>CH-), 1.87(q,2H,³J_{HH}=7Hz,-CH₂-), 3.65(dd,2H,²J_{HH}-14Hz, ³J_{HH}=7Hz,-CHH'O-), 3.70(dd,2H,²J_{HH}=14Hz,³J_{HH}=7Hz, -CHH'O-), 4.05(s,3H,-OCH₃), 4.10(t,2H,³J_{HH}=7Hz,>NCH₂), 7.80(s,1H,H-8).

### e) 9-(4-Hydroxy-3-hydroxymethylbut-1-yl)guanine

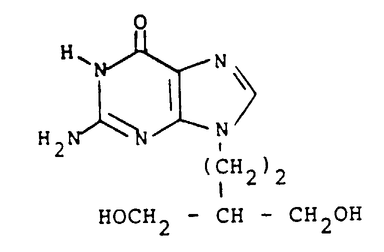

2-Amino-6-methoxy-9-(4-hydroxy-3-hydroxymethylbut-1-yl) purine (360mg, 1.35mmol) was dissolved in 2M aqueous sodium hydroxide solution (20ml) and the mixture heated at 80°C for 2h. The mixture was cooled, neutralized using 2M aqueous hydrochloric acid and evaporated down to a volume of approximately 20ml under reduced pressure then left to stand at 4°C for 18h. The resulting precipitate was filtered off to give a white powder (260mg, 76%), a sample of which was recrystallized from water to give colourless crystals.

m.p. 275-277°C.

¹H n.m.r.(D⁶-DMSO):δ1.50(m,1H,>CH-), 1.75(q,2H,³J_{HH}=7Hz,-CH₂-), 3.43(m,4H,-CH₂O-), 4.40(t,2H,³J_{HH}=7Hz,>NCH₂-),6.40(brs,2H,-NH₂), 7.70(s,1H,H-8),10.50(brs,1H,H-1).

## Claims (Claims for the following Contracting State(s): BE, CH, DE, FR, GB, IT, LI, NL)

1. A process for the preparation of a compound of formula (A): 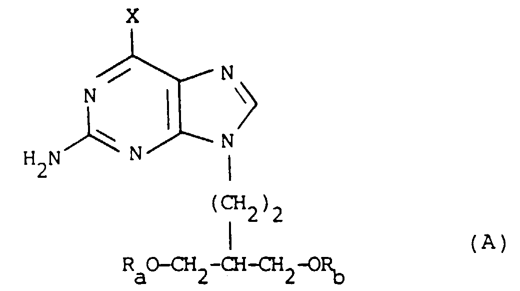 wherein:
X is hydrogen, hydroxy, chloro, C₁₋₆ alkoxy or phenyl C₁₋₆ alkoxy; and Rₐ and R_{b} are hydrogen, or acyl or phosphate derivatives thereof, which process comprises:
(i) the preparation of a compound of formula (I): 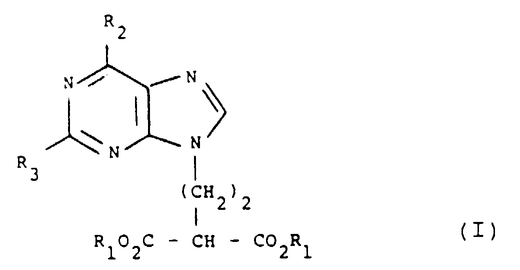 wherein R₁ is C₁₋₆ alkyl, or phenyl C₁₋₆ alkyl in which the phenyl group is optionally substituted by one or two substituents selected from hydroxy, C₁₋₆ alkyl, C₁₋₆ alkoxy and halogen; R₂ is hydrogen, hydroxy, chlorine, C₁₋₆ alkoxy, phenyl C₁₋₆ alkoxy or amino; and R₃ is halogen, C₁₋₆ alkylthio, C₁₋₆ alkylsulphonyl, azido, an amino group or a protected amino group, which preparation comprises the reaction of a compound of formula (II): 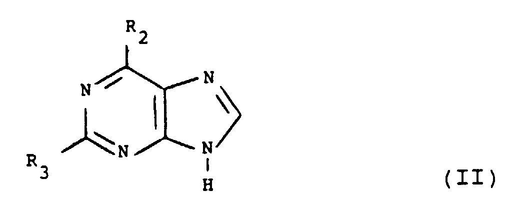 wherein R₂ and R₃ are as defined for formula (I) with:
(a), a compound of formula (III): 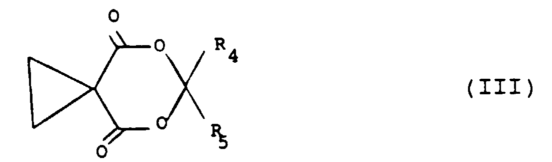 wherein R₄ and R₅ are independently hydrogen, C₁₋₆ alkyl, or phenyl, or R₄ and R₅ together are C₅₋₇ cycloalkyl, to give a compound of formula (IV): 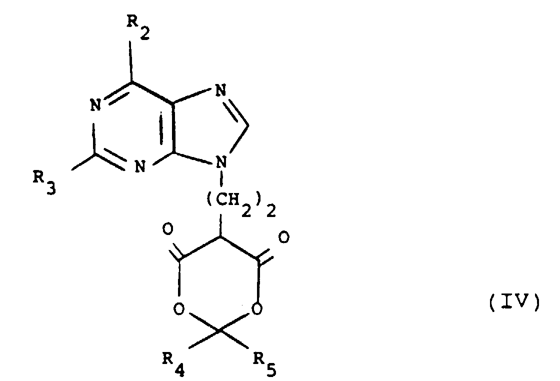 or
(b), a compound of formula (V): 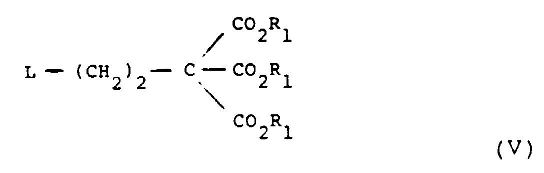 wherein L is a leaving group and R₁ is as defined for formula (I), to give a compound of formula (VI): 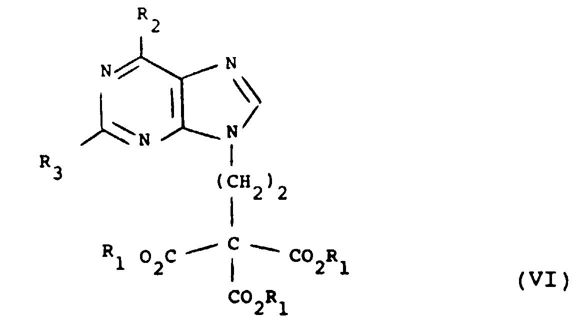 and thereafter converting the intermediate compound of formula (IV) to a compound of formula (I) via transesterification, or the intermediate compound of formula (VI) to a compound of formula (I) via decarboxylation, and, as necessary or desired, interconverting variables R₁, R₂ and R₃ to further values of R₁, R₂ and R₃;
(ii) the conversion of the resulting compound of formula (I) to a compound of formula (A) by converting variable R₃, when other than amino, to amino, reducing the ester groups CO₂R₁ to CH₂OH and optionally forming acyl or phosphate derivatives thereof, and as necessary or desired converting variable R₂ in the compound of formula (I) to variable X in the compound of formula (A).

2. A process for the preparation of a compound of formula (I) as defined in claim 1 by process step variant (a) as defined in claim l, which comprises the reaction of a compound of formula (II) wherein R₂ and R₃ are as defined in claim 1 with a compound of formula (III) wherein one of R₄ and R₅ is hydrogen and the other is methyl or phenyl, both of R₄ and R₅ are methyl or R₄ and R₅ together are cyclohexyl, followed by transesterification of the resulting compound of formula (IV) by reaction with an alcohol of formula (VII):
R₁-OH (VII)
wherein:
R₁ is C₁₋₄ alkyl, and, as necessary or desired, interconverting R₁, R₂ and R₃ in the resulting compound of formula (I) to further values of R₁, R₂ and R₃ as defined for formula (I) in claim 1.

3. A process for the preparation of a compound of formula (I) as defined in claim 1 by process step variant (b) as defined in claim 1, which comprises the reaction of a compound of formula (II) wherein R₂ and R₃ are as defined in claim 1 with a compound of formula (V) wherein R₁ is C₁₋₄ alkyl and L is halogen, followed by decarboxylation of the resulting compound of formula (VI), and, as necessary or desired, interconverting R₁, R₂ and R₃ in the resulting compound of formula (I) to further values of R₁, R₂ and R₃ as defined for formula (I) in claim 1.

4. A compound of formula (I) or a salt thereof: 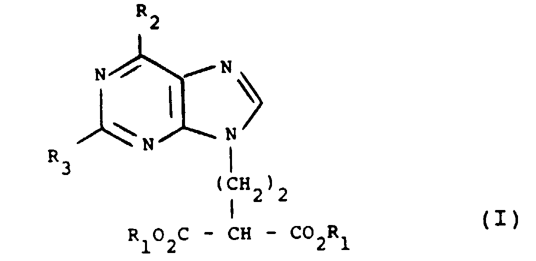 wherein R₁, R₂ and R₃ are as defined in claim 1.

5. A compound according to claim 4 or a salt thereof, wherein R₁ is methyl or ethyl; R₂ is hydrogen or methoxy; and R₃ is amino.

6. A compound of formula (IV) or a salt thereof: 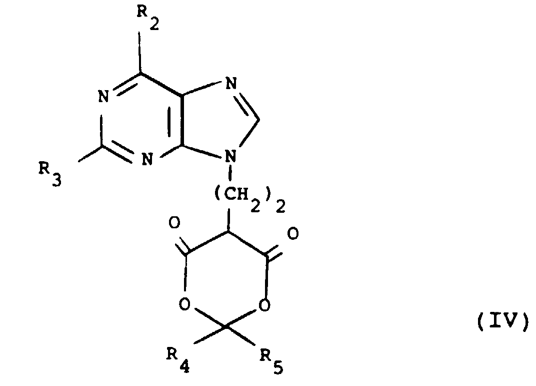 wherein:
R₂, R₃, R₄ and R₅ are as defined in claim 1.

7. A compound according to claim 6 or a salt thereof, wherein R₂ is hydrogen or chlorine; R₃ is amino, acetylamino or chlorine; one of R₄ and R₅ is hydrogen and the other is phenyl, R₄ and R₅ are both methyl, or R₄ and R₅ together are cyclohexyl.

8. A compound of formula (VI) or a salt thereof: 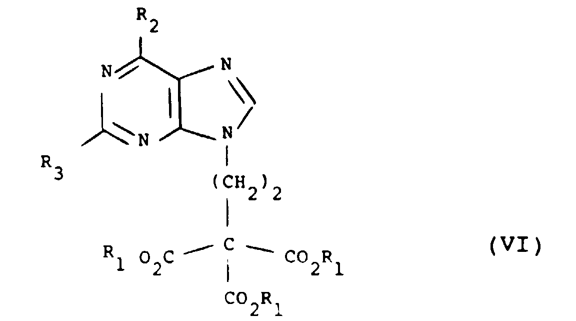 wherein:
R₁, R₂ and R₃ are as defined in claim 1.

9. A compound according to claim 8 or a salt thereof, wherein R₁ is ethyl; R₂ is hydrogen or chlorine and R₃ is amino.

## Claims (Claims for the following Contracting State(s): ES)

1. A process for the preparation of a compound of formula (A): 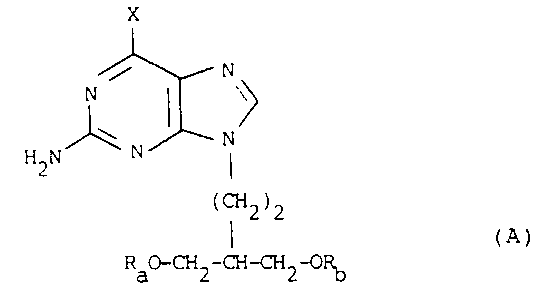 wherein:
X is hydrogen, hydroxy, chloro, C₁₋₆ alkoxy or phenyl C₁₋₆ alkoxy; and Rₐ and R_{b} are hydrogen, or acyl or phosphate derivatives thereof, which process comprises:
(i) the preparation of a compound of formula (I): 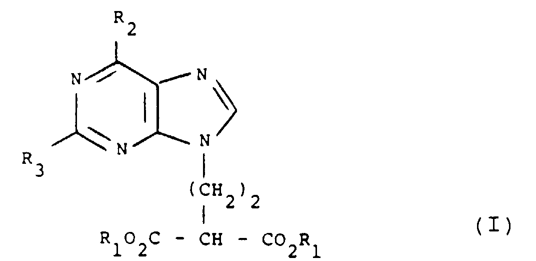 wherein R₁ is C₁₋₆ alkyl, or phenyl C₁₋₆ alkyl in which the phenyl group is optionally substituted by one or two substituents selected from hydroxy, C₁₋₆ alkyl, C₁₋₆ alkoxy and halogen; R₂ is hydrogen, hydroxy, chlorine, C₁₋₆ alkoxy, phenyl C₁₋₆ alkoxy or amino; and R₃ is halogen, C₁₋₆ alkylthio, C₁₋₆ alkylsulphonyl, azido, an amino group or a protected amino group, which preparation comprises the reaction of a compound of formula (II): 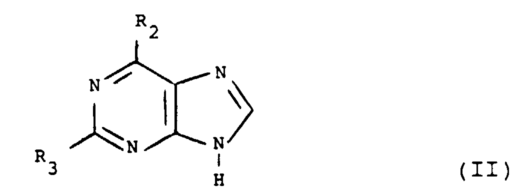 wherein R₂ and R₃ are as defined for formula (I) with:
(a), a compound of formula (III): 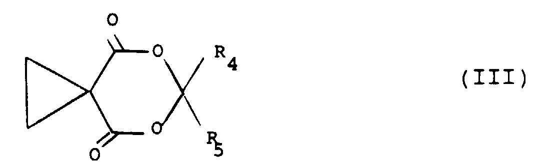 wherein R₄ and R₅ are independently hydrogen, C₁₋₆ alkyl, or phenyl, or R₄ and R₅ together are C₅₋₇ cycloalkyl, to give a compound of formula (IV): 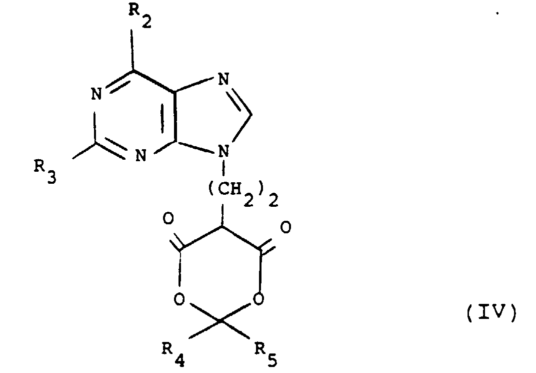 or
(b), a compound of formula (V): 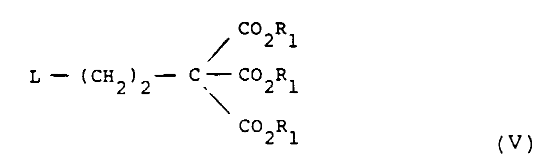 wherein L is a leaving group and R₁ is as defined for formula (I), to give a compound of formula (VI): 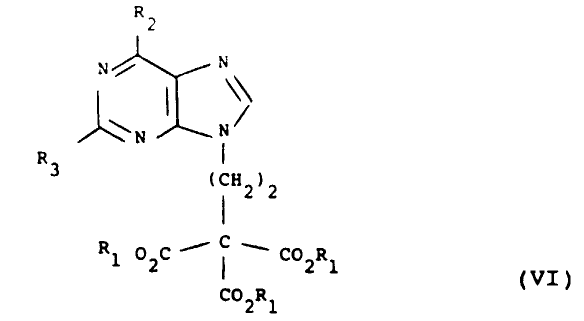 and thereafter converting the intermediate compound of formula (IV) to a compound of formula (I) via transesterification, or the intermediate compound of formula (VI) to a compound of formula (I) via decarboxylation, and, as necessary or desired, interconverting variables R₁, R₂ and R₃ to further values of R₁, R₂ and R₃;
(ii) the conversion of the resulting compound of formula (I) to a compound of formula (A) by converting variable R₃, when other than amino, to amino, reducing the ester groups CO₂R₁ to CH₂OH and optionally forming acyl or phosphate derivatives thereof, and as necessary or desired converting variable R₂ in the compound of formula (I) to variable X in the compound of formula (A).

2. A process for the preparation of a compound of formula (I) as defined in claim 1 by process step variant (a) as defined in claim 1, which comprises the reaction of a compound of formula (II) wherein R₂ and R₃ are as defined in claim 1 with a compound of formula (III) wherein one of R₄ and R₅ is hydrogen and the other is methyl or phenyl, both of R₄ and R₅ are methyl or R₄ and R₅ together are cyclohexyl, followed by transesterification of the resulting compound of formula (IV) by reaction with an alcohol of formula (VII):
R₁-OH (VII)
wherein:
R₁ is C₁₋₄ alkyl, and, as necessary or desired, interconverting R₁, R₂ and R₃ in the resulting compound of formula (I) to further values of R₁, R₂ and R₃ as defined for formula (I) in claim 1.

3. A process for the preparation of a compound of formula (I) as defined in claim 1 by process step variant (b) as defined in claim 1, which comprises the reaction of a compound of formula (II) wherein R₂ and R₃ are as defined in claim 1 with a compound of formula (V) wherein R₁ is C₁₋₄ alkyl and L is halogen, followed by decarboxylation of the resulting compound of formula (VI), and, as necessary or desired, interconverting R₁, R₂ and R₃ in the resulting compound of formula (I) to further values of R₁, R₂ and R₃ as defined for formula (I) in claim 1.

4. A process according to claim 1 for the preparation of a compound of formula (I) or a salt thereof: 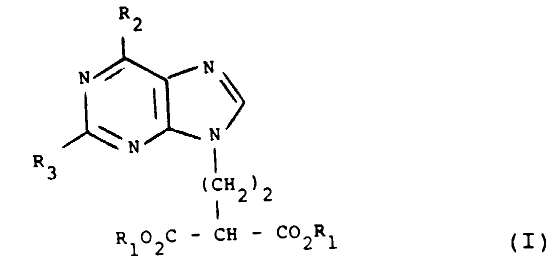 wherein R₁, R₂ and R₃ are as defined in claim 1.

5. A process according to claim 2 or 3 for the preparation of a compound of formula (I) or a salt thereof, wherein R₁ is methyl or ethyl; R₂ is hydrogen or methoxy; and R₃ is amino.

6. A process according to claim 1 for the preparation of a compound of formula (IV) or a salt thereof: 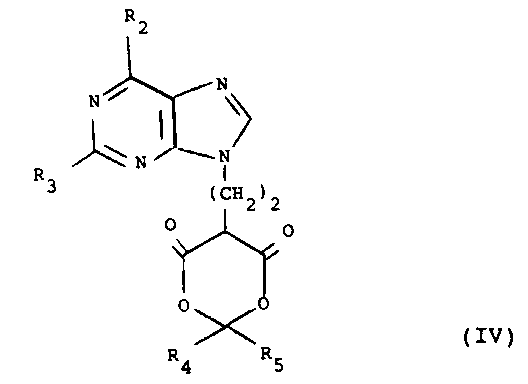 wherein:
R₂, R₃, R₄ and R₅ are as defined in claim 1.

7. A process according to claim 6 for the preparation of a compound of formula (IV) or a salt thereof, wherein R₂ is hydrogen or chlorine; R₃ is amino, acetylamino or chlorine; one of R₄ and R₅ is hydrogen and the other is phenyl, R₄ and R₅ are both methyl, or R₄ and R₅ together are cyclohexyl.

8. A process according to claim 1 for the preparation of a compound of formula (VI) or a salt thereof: 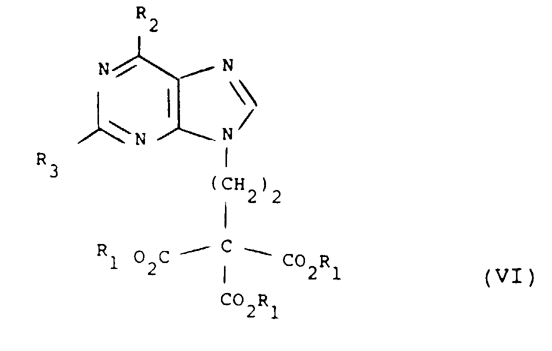 wherein:
R₁, R₂ and R₃ are as defined in claim 1.

9. A process according to claim 8 for the preparation of a compound of formula (VI) or a salt thereof, wherein R₁ is ethyl; R₂ is hydrogen or chlorine and R₃ is amino.

## Patentansprüche (Patentansprüche für folgende(n) Vertragsstaat(en): BE, CH, DE, FR, GB, IT, LI, NL)

1. Verfahren zur Herstellung einer Verbindung der Formel (A): 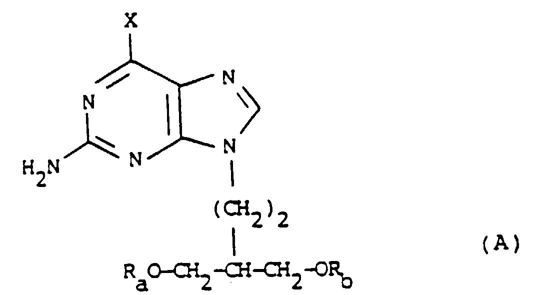 in der:
X ein Wasserstoffatom, eine Hydroxygruppe, ein Chloratom, einen C₁₋₆-Alkoxy- oder Phenyl-C₁₋₆-alkoxyrest bedeutet und Rₐ und R_{b} ein Wasserstoffatom darstellen, oder Acyl- oder Phosphatderivate davon, wobei das Verfahren:
(i) die Herstellung einer Verbindung der Formel (I): 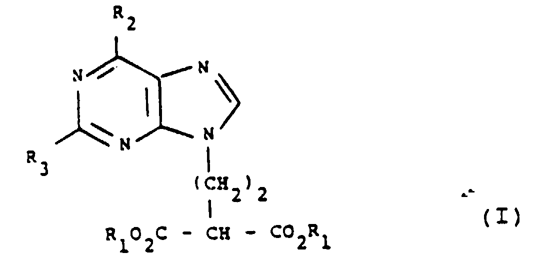 in der R₁ einen C₁₋₆-Alkyl- oder Phenyl-C₁₋₆-alkylrest bedeutet, bei dem die Phenylgruppe gegebenenfalls mit einem oder zwei Substituenten, die aus einer Hydroxygruppe, einem C₁₋₆-Alkylrest, einem C₁₋₆-Alkoxyrest und einem Halogenatom ausgewählt werden, substituiert ist, R₂ ein Wasserstoffatom, eine Hydroxygruppe, ein Chloratom, einen C₁₋₆-Alkoxyrest, einen Phenyl-C₁₋₆-alkoxyrest oder eine Aminogruppe darstellt und R₃ ein Halogenatom, einen C₁₋₆-Alkylthiorest, einen C₁₋₆-Alkylsulfonylrest, eine Azidogruppe, eine Aminogruppe oder eine geschützte Aminogruppe bedeutet, wobei die Herstellung die Umsetzung einer Verbindung der Formel (II) umfaßt : 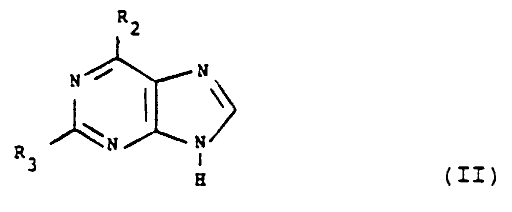 in der R₂ und R₃ wie für Formel (I) definiert sind, mit:
(a) einer Verbindung der Formel (III): 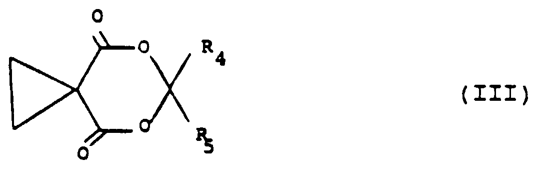 in der R₄ und R₅ unabhängig voneinander ein Wasserstoffatom, einen C₁₋₆-Alkylrest oder eine Phenylgruppe bedeuten oder R₄ und R₅ zusammen einen C₅₋₇-Cycloalkylrest darstellen, wobei eine Verbindung der Formel (IV): 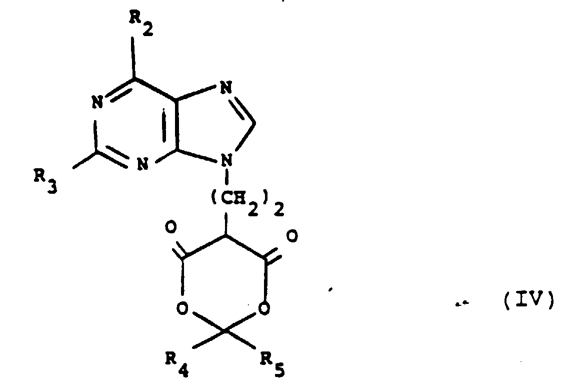 erhalten wird, oder
(b) einer Verbindung der Formel (V): 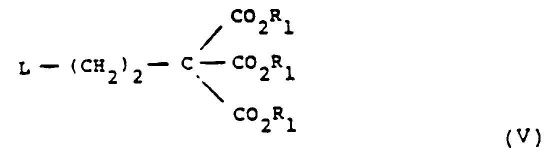 in der L eine Abgangsgruppe bedeutet und R₁ wie für Formel (I) definiert ist, wobei eine Verbindung der Formel (VI): 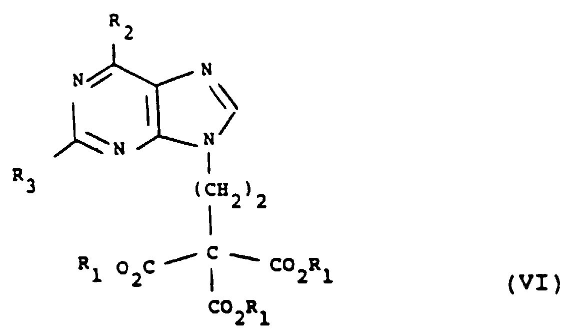 erhalten wird, und anschließendes Umwandeln der Zwischenverbindung der Formel (IV) über Umesterung zu einer Verbindung der Formel (I) oder der Zwischenverbindung der Formel (VI) über Decarboxylierung zu einer Verbindung der Formel (I) und, falls notwendig oder erwünscht, wechselseitiges Umwandeln der Variablen R₁, R₂ und R₃ zu weiteren Werten von R₁, R₂ und R₃ umfaßt;
(ii) die Umwandlung der erhaltenen Verbindung der Formel (I) zu einer Verbindung der Formel (A) durch Umwandeln der Variablen R₃, wenn sie keine Aminogruppe darstellt, zu einer Aminogruppe, Reduzieren der Estergruppen CO₂R₁ zu CH₂OH und gegebenenfalls Bilden von Acyl- oder Phosphatderivaten davon und, falls notwendig oder erwünscht, Umwandeln der Variablen R₂ in der Verbindung der Formel (I) zur Variablen X in der Verbindung der Formel (A) umfaßt.

2. Verfahren zur Herstellung einer Verbindung der Formel (I) nach Anspruch 1 durch Verfahrensschrittvariante (a) nach Anspruch 1, das die Umsetzung einer Verbindung der Formel (II), in der R₂ und R₃ wie in Anspruch 1 definiert sind, mit einer Verbindung der Formel (III), in der einer der Reste R₄ und R₅ ein Wasserstoffatom bedeutet und der andere eine Methyl- oder Phenylgruppe darstellt, beide Reste R₄ und R₅ eine Methylgruppe bedeuten oder R₄ und R₅ zusammen eine Cyclohexylgruppe darstellen, gefolgt von einer Umesterung der erhaltenen Verbindung der Formel (IV) durch Umsetzung mit einem Alkohol der Formel (VII):
R₁-OH (VII)
in der:
R₁ einen C₁₋₄-Alkylrest bedeutet, und, falls notwendig oder erwünscht, wechselseitiges Umwandeln von R₁, R₂ und R₃ in der erhaltenen Verbindung der Formel (I) zu weiteren Werten von R₁, R₂ und R₃, wie für Formel (I) in Anspruch 1 definiert, umfaßt.

3. Verfahren zur Herstellung einer Verbindung der Formel (I) nach Anspruch 1 durch Verfahrensschrittvariante (b) nach Anspruch 1, das die Umsetzung einer Verbindung der Formel (II), in der R₂ und R₃ wie in Anspruch 1 definiert sind, mit einer Verbindung der Formel (V), in der R₁ einen C₁₋₄-Alkylrest bedeutet und L ein Halogenatom darstellt, gefolgt von einer Decarboxylierung der erhaltenen Verbindung der Formel (VI), und, falls notwendig oder erwünscht, wechselseitiges Umwandeln von R₁, R₂ und R₃ in der erhaltenen Verbindung der Formel (I) zu weiteren Werten von R₁, R₂ und R₃, wie für Formel (I) in Anspruch 1 definiert, umfaßt.

4. Verbindung der Formel (I) oder ein Salz davon: 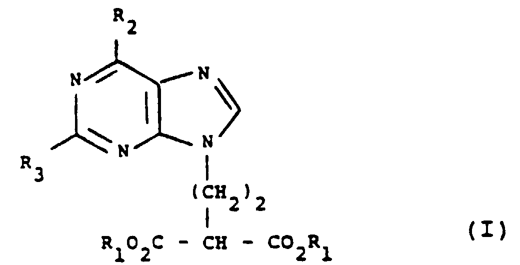 in der R₁, R₂ und R₃ wie in Anspruch 1 definiert sind.

5. Verbindung nach Anspruch 4 oder ein Salz davon, in der R₁ eine Methyl- oder Ethylgruppe darstellt, R₂ ein Wasserstoffatom oder eine Methoxygruppe bedeutet und R₃ eine Aminogruppe darstellt.

6. Verbindung der Formel (IV) oder ein Salz davon: 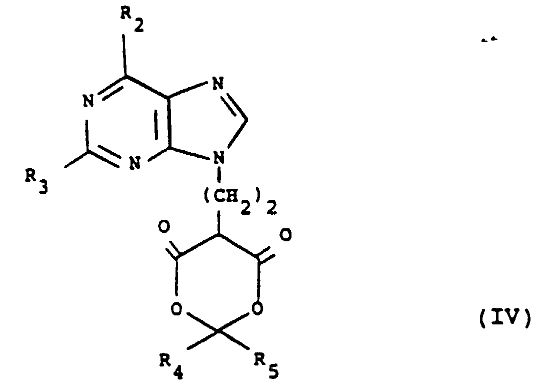 in der:
R₂, R₃, R₄ und R₅ wie in Anspruch 1 definiert sind.

7. Verbindung nach Anspruch 6 oder ein Salz davon, in der R₂ ein Wasserstoffoder Chloratom bedeutet. R₃ eine Aminogruppe, eine Acetylaminogruppe oder ein Chloratom darstellt, einer der Reste R₄ und R₅ ein Wasserstoffatom bedeutet und der andere eine Phenylgruppe darstellt. R₄ und R₅ beide eine Methylgruppe bedeuten oder R₄ und R₅ zusammen eine Cyclohexylgruppe darstellen.

8. Verbindung der Formel (VI) oder ein Salz davon: 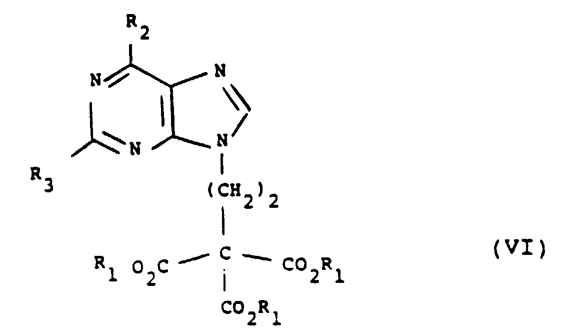 in der:
R₁, R₂ und R₃ wie in Anspruch 1 definiert sind.

9. Verbindung nach Anspruch 8 oder ein Salz davon, in der R₁ eine Ethylgruppe darstellt, R₂ ein Wasserstoff- oder Chloratom bedeutet und R₃ eine Aminogruppe darstellt.

## Patentansprüche (Patentansprüche für folgende(n) Vertragsstaat(en): ES)

1. Verfahren zur Herstellung einer Verbindung der Formel (A): 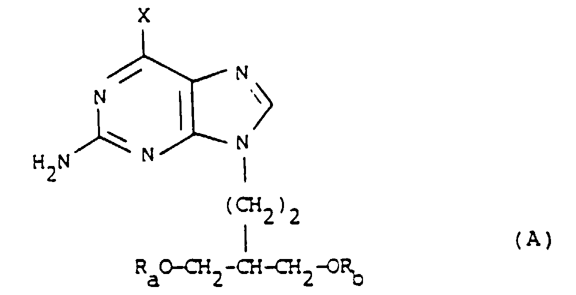 in der:
X ein Wasserstoffatom, eine Hydroxygruppe, ein Chloratom, einen C₁₋₆-Alkoxy- oder Phenyl-C₁₋₆-alkoxyrest bedeutet und Rₐ und R_{b} ein Wasserstoffatom darstellen, oder Acyl- oder Phosphatderivate davon, wobei das Verfahren:
(i) die Herstellung einer Verbindung der Formel (I): 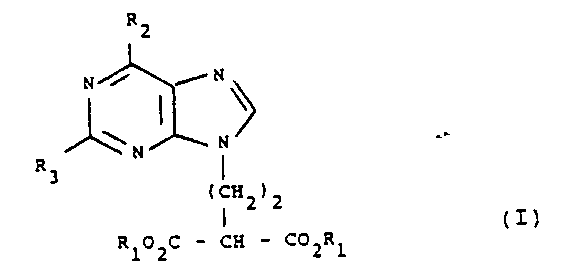 in der R₁ einen C₁₋₆-Alkyl- oder Phenyl-C₁₋₆-alkylrest bedeutet, bei dem die Phenylgruppe gegebenenfalls mit einem oder zwei Substituenten, die aus einer Hydroxygruppe, einem C₁₋₆-Alkylrest, einem C₁₋₆-Alkoxyrest und einem Halogenatom ausgewählt werden, substituiert ist, R₂ ein Wasserstoffatom, eine Hydroxygruppe, ein Chloratom, einen C₁₋₆-Alkoxyrest, einen Phenyl-C₁₋₆-alkoxyrest oder eine Aminogruppe darstellt und R3 ein Halogenatom, einen C₁₋₆-Alkylthiorest, einen C₁₋₆-Alkylsulfonylrest, eine Azidogruppe, eine Aminogruppe oder eine geschützte Aminogruppe bedeutet, wobei die Herstellung die Umsetzung einer Verbindung der Formel (II) umfaßt : 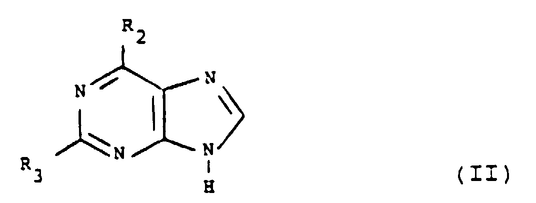 in der R₂ und R₃ wie für Formel (I) definiert sind. mit:
(a) einer Verbindung der Formel (III): 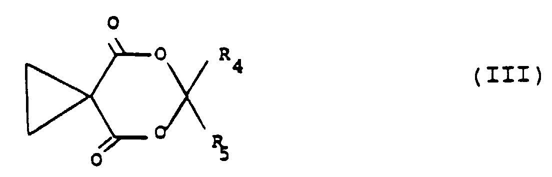 in der R₄ und R₅ unabhängig voneinander ein Wasserstoffatom, einen C₁₋₆-Alkylrest oder eine Phenylgruppe bedeuten oder R₄ und R₅ zusammen einen C₅₋₇-Cycloalkylrest darstellen, wobei eine Verbindung der Formel (IV) erhalten wird: 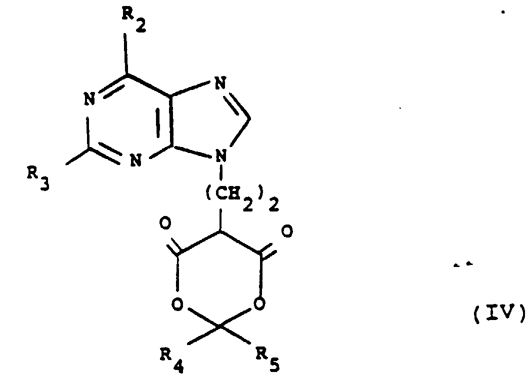 oder
(b) einer Verbindung der Formel (V): 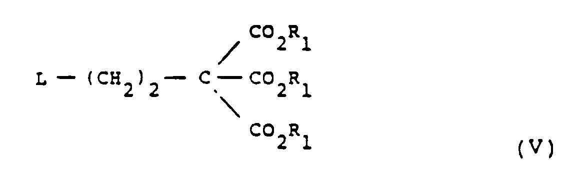 in der L eine Abgangsgruppe bedeutet und R₁ wie für Formel (I) definiert ist, wobei eine Verbindung der Formel (VI): 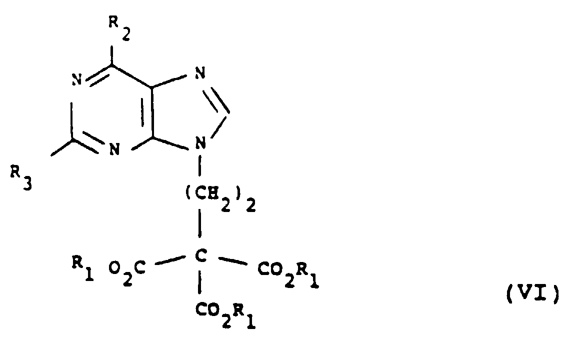 erhalten wird, und anschließendes Umwandeln der Zwischenverbindung der Formel (IV) über Umesterung zu einer Verbindung der Formel (I) oder der Zwischenverbindung der Formel (VI) über Decarboxylierung zu einer Verbindung der Formel (I) und, falls notwendig oder erwünscht, wechselseitiges Umwandeln der Variablen R₁, R₂ und R₃ zu weiteren Werten von R₁, R₂ und R₃ umfaßt;
(ii) die Umwandlung der erhaltenen Verbindung der Formel (I) zu einer Verbindung der Formel (A) durch Umwandeln der Variablen R₃, wenn sie keine Aminogruppe darstellt, zu einer Aminogruppe, Reduzieren der Estergruppen CO₂R₁ zu CH₂OH und gegebenenfalls Bilden von Acyl- oder Phosphatderivaten davon und, falls notwendig oder erwünscht, Umwandeln der Variablen R₂ in der Verbindung der Formel (I) zur Variablen X in der Verbindung der Formel (A) umfaßt.

2. Verfahren zur Herstellung einer Verbindung der Formel (I) nach Anspruch 1 durch Verfahrensschrittvariante (a) nach Anspruch 1, das die Umsetzung einer Verbindung der Formel (II), in der R₂ und R₃ wie in Anspruch 1 definiert sind, mit einer Verbindung der Formel (III), in der einer der Reste R₄ und R₅ ein Wasserstoffatom bedeutet und der andere eine Methyl- oder Phenylgruppe darstellt, beide Reste R₄ und R₅ eine Methylgruppe bedeuten oder R₄ und R₅ zusammen eine Cyclohexylgruppe darstellen, gefolgt von einer Umesterung der erhaltenen Verbindung der Formel (IV) durch Umsetzung mit einem Alkohol der Formel (VII):
R₁-OH (VII)
in der:
R₁ einen C₁₋₄-Alkylrest bedeutet, und, falls notwendig oder erwünscht, wechselseitiges Umwandeln von R₁, R₂ und R₃ in der erhaltenen Verbindung der Formel (I) zu weiteren Werten von R₁, R₂ und R₃, wie für Formel (I) in Anspruch 1 definiert, umfaßt.

3. Verfahren zur Herstellung einer Verbindung der Formel (I) nach Anspruch 1 durch Verfahrensschrittvariante (b) nach Anspruch 1, das die Umsetzung einer Verbindung der Formel (II), in der R₂ und R₃ wie in Anspruch 1 definiert sind, mit einer Verbindung der Formel (V), in der R₁ einen C₁₋₄-Alkylrest bedeutet und L ein Halogenatom darstellt, gefolgt von einer Decarboxylierung der erhaltenen Verbindung der Formel (VI), und, falls notwendig oder erwünscht, wechselseitiges Umwandeln von R₁, R₂ und R₃ in der erhaltenen Verbindung der Formel (I) zu weiteren Werten von R₁, R₂ und R₃, wie für Formel (I) in Anspruch 1 definiert, umfaßt.

4. Verfahren nach Anspruch 1 zur Herstellung einer Verbindung der Formel (I) oder eines Salzes davon: 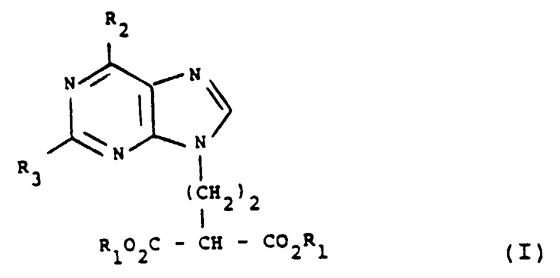 wobei R₁, R₂ und R₃ wie in Anspruch 1 definiert sind.

5. Verfahren nach Anspruch 2 oder 3 zur Herstellung einer Verbindung der Formel (I) oder eines Salzes davon, wobei R₁ eine Methyl- oder Ethylgruppe darstellt, R₂ ein Wasserstoffatom oder eine Methoxygruppe bedeutet und R₃ eine Aminogruppe darstellt.

6. Verfahren nach Anspruch 1 zur Herstellung einer Verbindung der Formel (IV) oder eines Salzes davon: 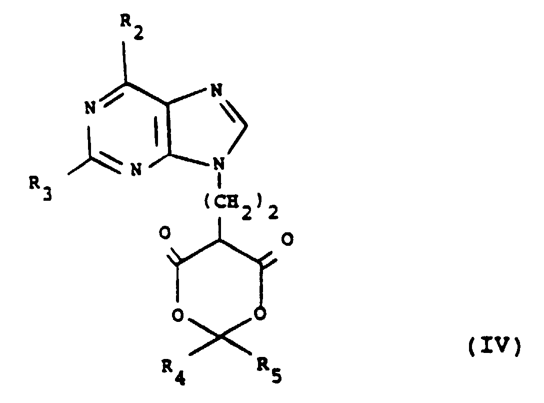 wobei:
R₂, R₃, R₄ und R₅ wie in Anspruch 1 definiert sind.

7. Verfahren nach Anspruch 6 zur Herstellung einer Verbindung der Formel (IV) oder eines Salzes davon. wobei R₂ ein Wasserstoff- oder Chloratom bedeutet. R₃ eine Aminogruppe, eine Acetylaminogruppe oder ein Chloratom darstellt, einer der Reste R4 und R₅ ein Wasserstoffatom bedeutet und der andere eine Phenylgruppe bedeutet, R₄ und R₅ beide eine Methylgruppe bedeuten oder R₄ und R₅ zusammen eine Cyclohexylgruppe darstellen.

8. Verfahren nach Anspruch 1 zur Herstellung einer Verbindung der Formel (VI) oder eines Salzes davon: 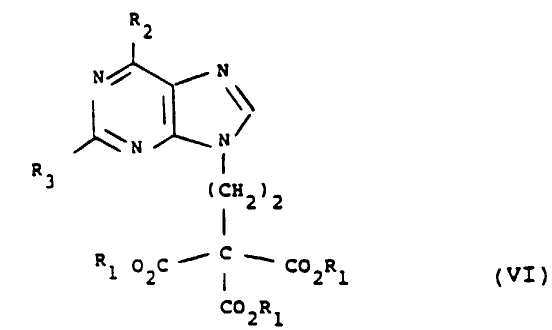 wobei:
R₁, R₂ und R₃ wie in Anspruch 1 definiert sind.

9. Verfahren nach Anspruch 8 zur Herstellung einer Verbindung der Formel (VI) oder eines Salzes davon, wobei R₁ eine Ethylgruppe darstellt, R₂ ein Wasserstoff- oder Chloratom bedeutet und R₃ eine Aminogruppe darstellt.

## Revendications (Revendications pour l'(les) Etat(s) contractant(s) suivant(s): BE, CH, DE, FR, GB, IT, LI, NL)

1. Procédé de préparation d'un composé de formule (A): 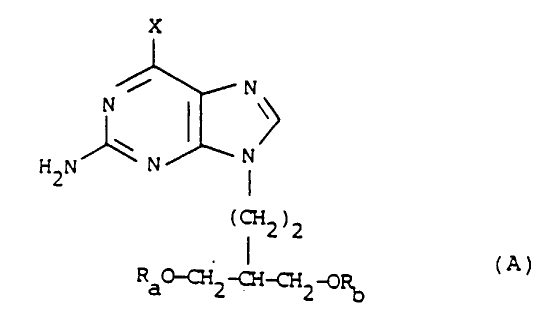 dans laquelle :
X représente un atome d'hydrogène, un groupe hydroxy, chloro, alcoxy C₁₋₆, ou phénylalcoxy C₁₋₆; et Rₐ et R_{b} représentent un atome d'hydrogène, ou des dérivés acyle ou phosphate de celui-ci, lequel procédé comprend :
(i) la préparation d'un composé de formule (I): 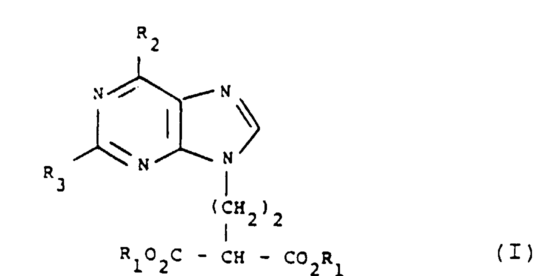 dans laquelle R₁ est un groupe alkyle C₁₋₆, ou phénylalkyle C₁₋₆ dans lequel le groupe phényle est éventuellement substitué par un ou deux substituants choisis parmi les groupes hydroxy, alkyle C₁₋₆, alcoxy C₁₋₆, et halogène ; R₂ est un atome d'hydrogène, un groupe hydroxy, chloro, alcoxy C₁₋₆, phénylalcoxy C₁₋₆, ou amino ; et R₃ est un halogène, un groupe alkylthio C₁₋₆, alkyl C₁₋₆ sulfonyle, azido, amino, ou un groupe amino protégé, laquelle préparation comprend la réaction d'un composé de formule (II) : 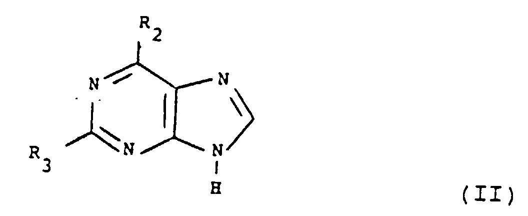 dans laquelle R₂ et R₃ sont tels que définis pour la formule (I), avec :
- (a) un composé de formule (III): 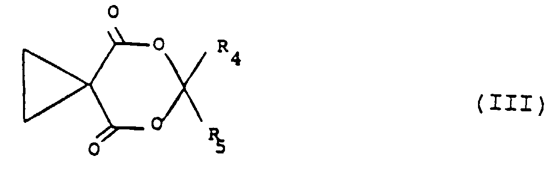 dans laquelle R₄ et R₅ sont indépendamment, un atome d'hydrogène, un groupe alkyle C₁₋₆, ou phényle, ou bien R₄ et R₅ ensemble sont un groupe cycloalkyle C₅₋₇, pour donner un composé de formule (IV) : 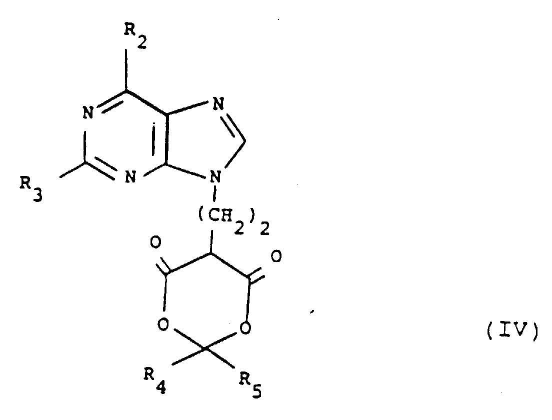 ou
- (b) un composé de formule (V) : 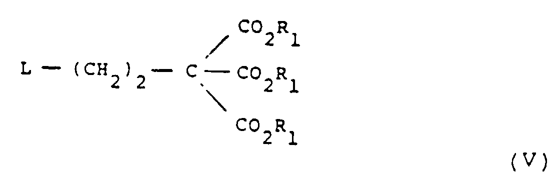 dans laquelle L est un groupe partant, et R₁ est tel que défini pour la formule (I), pour donner un composé de formule (VI) : 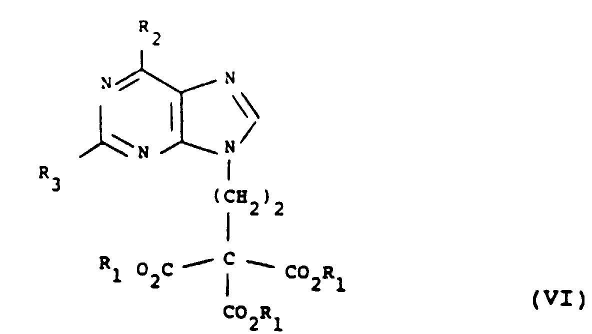 et ensuite, la conversion du produit intermédiaire de formule (IV), en un composé de formule (I) via une transestérification, ou du composé intermédiaire de formule (VI) en un composé de formule (I) via une décarboxylation, et si nécessaire ou désiré, l'interconversion des variables R₁, R₂, et R₃, en d'autres valeurs de R₁, R₂, et R₃ ;
(ii) la conversion du produit obtenu de formule (I) en un composé de formule (A) en convertissant la variable R₃, lorsqu'elle autre qu'un groupe amino, en un groupe amino, en réduisant les groupes ester COOR₁ en CH₂OH, et éventuellement en formant des dérivés acyle ou phosphate de celui-ci, et si nécessaire ou désiré, en convertissant la variable R₂ dans le composé de formule (I) en variable X dans le composé de formule (A).

2. Procédé de préparation d'un composé de formule (I) tel que défini dans la revendication 1, par la variante d'étape de procédé (a) telle que définie dans la revendication 1, lequel comprend la réaction d'un composé de formule (II) dans laquelle R₂ et R₃ sont tels que définis dans la revendication 1, avec un composé de formule (III) dans laquelle l'un des groupes R₄ ou R₅ est un atome d'hydrogène, et l'autre est un groupe méthyle ou phényle, ou R₄ et R₅ sont tous deux des groupes méthyle, ou R₄ et R₅ ensemble sont un groupe cyclohexyle, suivie de la transestérification du composé obtenu de formule (IV) par réaction avec un alcool de formule (VII) :
R₁-OH (VII)
dans laquelle :
R₁ est un groupe alkyle C₁₋₄, et si nécessaire ou désiré, en interconvertissant R₁, R₂, et R₃ dans le composé de formule (I) obtenu, en d'autres valeurs de R₁, R₂, et R₃ telles que définies pour la formule (I) dans la revendication 1.

3. Procédé de préparation d'un composé de formule (I) tel que défini dans la revendication 1, par la variante d'étape de procédé (b) telle que définie dans la revendication 1, lequel comprend la réaction d'un composé de formule (II) dans laquelle R₂ et R₃ sont tels que définis dans la revendication 1, avec un composé de formule (V) dans laquelle R₁ est un groupe alkyle C₁₋₄, et L est un halogène, suivie de la décarboxylation du composé de formule (VI) obtenu, et si nécessaire ou désiré, en interconvertissant R₁, R₂, et R₃ dans le composé de formule (I) obtenu, en d'autres valeurs de R₁, R₂, et R₃ telles que définies pour la formule (I) dans la revendication 1.

4. Composé de formule (I) ou sel de celui-ci : 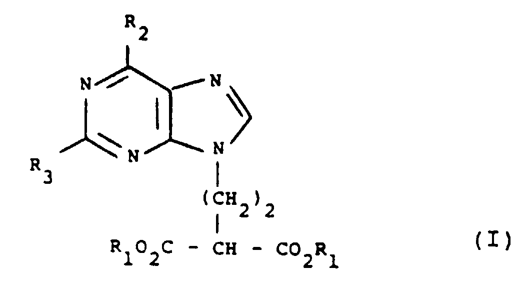 dans laquelle R₁, R₂, et R₃ telles que définies dans la revendication 1.

5. Composé selon la revendication 4, ou sel de celui-ci, dans lequel R₁ est un groupe méthyle ou éthyle; R₂ est un atome d'hydrogène ou un groupe méthoxy ; et R₃ est un groupe amino.

6. Composé de formule (IV) ou sel de celui-ci : 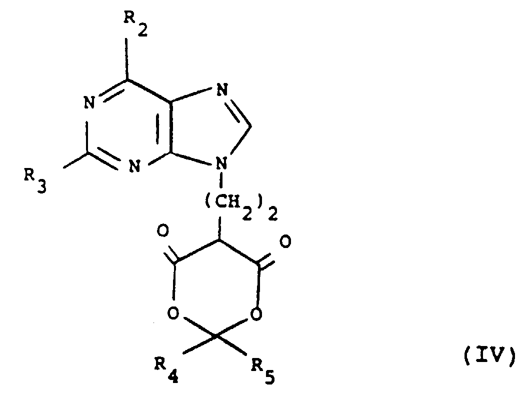 dans laquelle R₂, R₃, R₄, et R₅ sont tels que définis dans la revendication 1.

7. Composé selon la revendication 6, ou sel de celui-ci, dans lequel R₂ est un atome d'hydrogène ou de chlore ; R₃ est un groupe amino, acétylamino, ou chlore ; un des groupes R₄ ou R₅ est un atome d'hydrogène, et l'autre est un groupe phényle, ou R₄ et R₅ sont tous deux des groupes méthyle, ou R₄ et R₅ ensemble sont un groupe cyclohexyle.

8. Composé de formule (VI) ou sel de celui-ci : 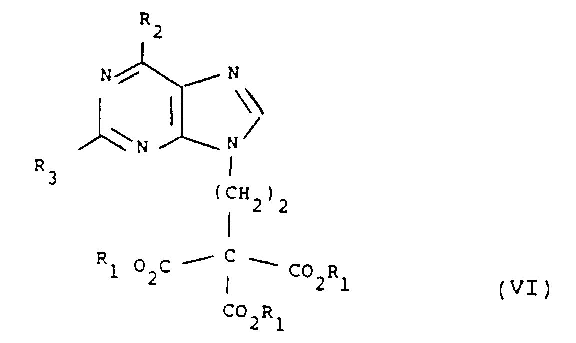 dans laquelle R₁, R₂, et R₃ sont tels que définis dans la revendication 1.

9. Composé selon la revendication 8, ou sel de celui-ci, dans lequel R₁ est un groupe éthyle ; R₂ est un atome d'hydrogène ou de chlore ; et R₃ est un groupe amino.

## Revendications (Revendications pour l'(les) Etat(s) contractant(s) suivant(s): ES)

1. Procédé de préparation d'un composé de formule (A) : 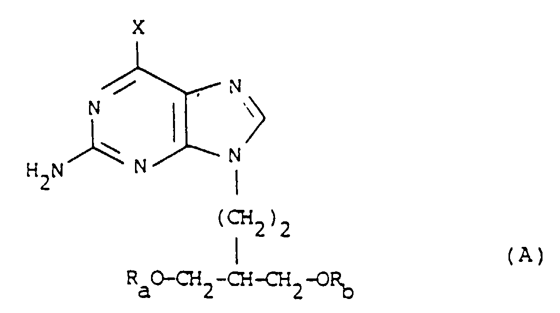 dans laquelle :
X représente un atome d'hydrogène, un groupe hydroxy, chloro, alcoxy C₁₋₆, ou phénylalcoxy C₁₋₆; et Rₐ et R_{b} représentent un atome d'hydrogène, ou des dérivés acyle ou phosphate de celui-ci, lequel procédé comprend :
(i) la préparation d'un composé de formule (I) : 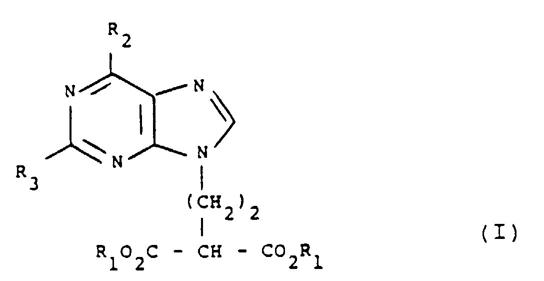 dans laquelle R₁ est un groupe alkyle C₁₋₆, ou phénylalkyle C₁₋₆ dans lequel le groupe phényle est éventuellement substitué par un ou deux substituants choisis parmi les groupes hydroxy, alkyle C₁₋₆, alcoxy C₁₋₆, et halogène ; R₂ est un atome d'hydrogène, un groupe hydroxy, chloro, alcoxy C₁₋₆, phénylalcoxy C₁₋₆, ou amino; et R₃ est un halogène, un groupe alkylthio C₁₋₆, alkyl C₁₋₆ sulfonyle, azido, amino, ou un groupe amino protégé, laquelle préparation comprend la réaction d'un composé de formule (II): 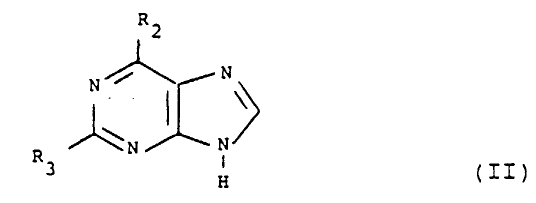 dans laquelle R₂ et R₃ sont tels que définis pour la formule (I), avec :
- (a) un composé de formule (III): 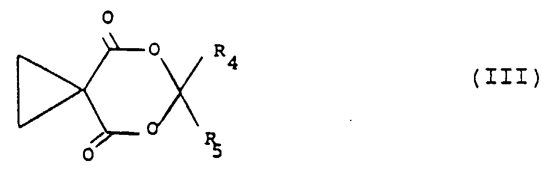 dans laquelle R₄ et R₅ sont indépendamment, un atome d'hydrogène, un groupe alkyle C₁₋₆, ou phényle, ou bien R₄ et R₅ ensemble sont un groupe cycloalkyle C₅₋₇, pour donner un composé de formule (IV) : 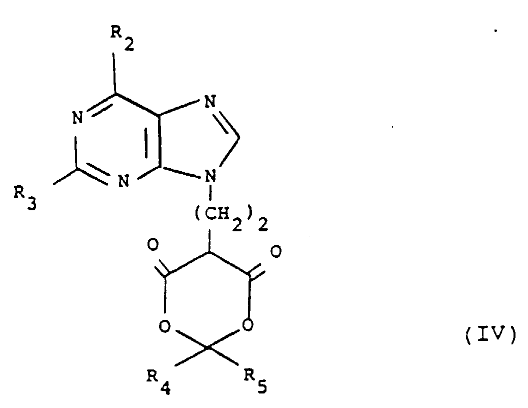 ou
- (b) un composé de formule (V) : 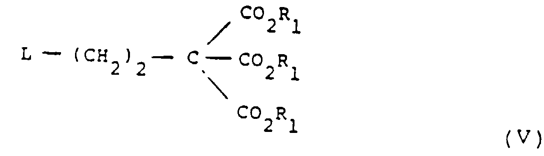 dans laquelle L est un groupe partant, et R₁ est tel que défini pour la formule (I), pour donner un composé de formule (VI) : 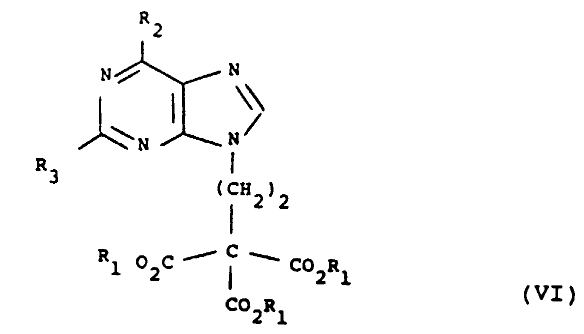 et ensuite, la conversion du produit intermédiaire de formule (IV), en un composé de formule (I) via une transestérification, ou du composé intermédiaire de formule (VI) en un composé de formule (I) via une décarboxylation, et si nécessaire ou désiré, l'interconversion des variables R₁, R₂, et R₃, en d'autres valeurs de R₁, R₂, et R₃ ;
(ii) la conversion du produit obtenu de formule (I) en un composé de formule (A) en convertissant la variable R₃, lorsqu'elle autre qu'un groupe amino, en un groupe amino, en réduisant les groupes ester COOR₁ en CH₂OH, et éventuellement en formant des dérivés acyle ou phosphate de celui-ci, et si nécessaire ou désiré, en convertissant la variable R₂ dans le composé de formule (I) en variable X dans le composé de formule (A).

2. Procédé de préparation d'un composé de formule (I) tel que défini dans la revendication 1, par la variante d'étape de procédé (a) telle que définie dans la revendication 1, lequel comprend la réaction d'un composé de formule (II) dans laquelle R₂ et R₃ sont tels que définis dans la revendication 1, avec un composé de formule (III) dans laquelle l'un des groupes R₄ ou R₅ est un atome d'hydrogène, et l'autre est un groupe méthyle ou phényle, ou R₄ et R₅ sont tous deux des groupes méthyle, ou R₄ et R₅ ensemble sont un groupe cyclohexyle, suivie de la transestérification du composé obtenu de formule (IV) par réaction avec un alcool de formule (VII):
R₁-OH (VII)
dans laquelle :
R₁ est un groupe alkyle C₁₋₄, et si nécessaire ou désiré, en interconvertissant R₁, R₂, et R₃ dans le composé de formule (I) obtenu, en d'autres valeurs de R₁, R₂, et R₃ telles que définies pour la formule (I) dans la revendication 1.

3. Procédé de préparation d'un composé de formule (I) tel que défini dans la revendication 1, par la variante d'étape de procédé (b) telle que définie dans la revendication 1, lequel comprend la réaction d'un composé de formule (II) dans laquelle R₂ et R₃ sont tels que définis dans la revendication 1, avec un composé de formule (V) dans laquelle R₁ est un groupe alkyle C₁₋₄, et L est un halogène, suivie de la décarboxylation du composé de formule (VI) obtenu, et si nécessaire ou désiré, en interconvertissant R₁, R₂, et R₃ dans le composé de formule (I) obtenu, en d'autres valeurs de R₁, R₂, et R₃ telles que définies pour la formule (I) dans la revendication 1.

4. Procédé selon la revendication 1, de préparation d'un composé de formule (I) ou d'un sel de celui-ci : 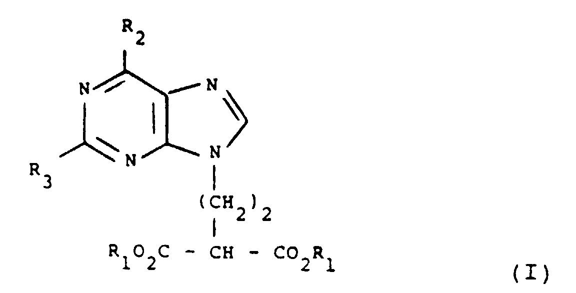 dans laquelle R₁, R₂, et R₃ telles que définies dans la revendication 1.

5. Procédé selon la revendication 2, ou la revendication 3, de préparation d'un composé de formule (I), ou d'un sel de celui-ci, dans laquelle R₁ est un groupe méthyle ou éthyle; R₂ est un atome d'hydrogène ou un groupe méthoxy; et R₃ est un groupe amino.

6. Procédé selon la revendication 1, de préparation d'un composé de formule (IV) ou d'un sel de celui-ci : 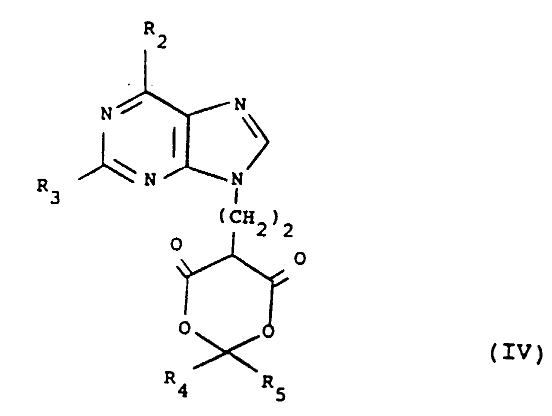 dans laquelle R₂, R₃, R₄, et R₅ sont tels que définis dans la revendication 1.

7. Procédé selon la revendication 6, de préparation d'un composé de formule (IV), ou d'un sel de celui-ci, dans laquelle R₂ est un atome d'hydrogène ou de chlore; R₃ est un groupe amino, acétylamino, ou chloro ; un des groupes R₄ ou R₅ est un atome d'hydrogène, et l'autre est un groupe phényle, ou R₄ et R₅ sont tous deux des groupes méthyle, ou R₄ et R₅ ensemble sont un groupe cyclohexyle.

8. Procédé selon la revendication 1, de préparation d'un composé de formule (VI) ou d'un sel de celui-ci : 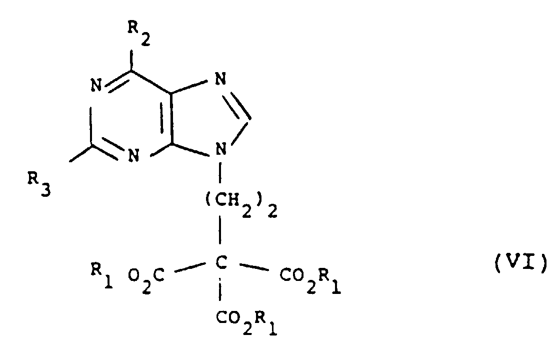 dans laquelle R₁, R₂, et R₃ sont tels que définis dans la revendication 1.

9. Procédé selon la revendication 8, de préparation d'un composé de formule (VI) ou d'un sel de celui-ci, dans laquelle R₁ est un groupe éthyle; R₂ est un atome d'hydrogène ou de chlore ; et R₃ est un groupe amino.
